(19) Europäisches Patentamt / European Patent Office / Office européen des brevets

(11) **EP 3 475 448 B1**

(12) **EUROPEAN PATENT SPECIFICATION**

(45) Date of publication and mention of the grant of the patent:
**30.03.2022 Bulletin 2022/13**

(21) Application number: **17816135.2**

(22) Date of filing: **21.06.2017**

(51) International Patent Classification (IPC):
**C12Q 1/6886** *(2018.01)*

(52) Cooperative Patent Classification (CPC):
**C12Q 1/6886;** C12Q 2600/156

(86) International application number:
**PCT/US2017/038533**

(87) International publication number:
**WO 2017/223194 (28.12.2017 Gazette 2017/52)**

(54) **METHOD AND KIT FOR DETECTING FUSION TRANSCRIPTS**

VERFAHREN UND KIT ZUR ERKENNUNG VON FUSIONSTRANSKRIPTEN

PROCÉDÉ ET KIT DE DÉTECTION DE PRODUITS DE TRANSCRIPTION DE FUSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2016 US 201615188982**

(43) Date of publication of application:
**01.05.2019 Bulletin 2019/18**

(73) Proprietor: **Zhuo, Degen**
**Palmetto Bay, FL 33157 (US)**

(72) Inventor: **Zhuo, Degen**
**Palmetto Bay, FL 33157 (US)**

(74) Representative: **Engelhard, Markus**
**Boehmert & Boehmert**
**Anwaltspartnerschaft mbB**
**Pettenkoferstrasse 22**
**80336 München (DE)**

(56) References cited:
US-A1- 2012 039 887    US-A1- 2013 059 299
US-A1- 2016 078 168    US-A1- 2016 078 168
US-A1- 2017 009 304    US-B1- 6 849 400

• ZHOU, JEFF XIWU ET AL.: 'identification of KANSARL as the first cancer predisposition fusion gene specific to the population of European ancestry origin' ONCOTARGET vol. 8, no. 31, 24 March 2017, pages 50594 - 50607, XP055448243

Remarks:
The complete document including Reference Tables and the Sequence Listing can be downloaded from the EPO website

## Description

REFERENCE TO SEQUENCE LISTING SUBMITTED ELECTRONICALLY

The content of the electronically submitted sequence listing, file name SeqList-20Jun17-ST25.txt, size 199,892 KB; and date of creation June 20, 2017, filed herewith, is incorporated herein by reference in its entirety.

BACKGROUND

Genetic predisposition to cancer has been well known for centuries initially via observation of unusual familial clustering of cancer, and later through identification of studying cancer-prone families that demonstrate Mendelian inheritance of cancer predisposition (Rahman 2014). 114 cancer predisposition genes (CPG) have been identified so far, including BRCA1 and BRCA2, the DNA-mismatch-repair genes (relevant for colon cancer), TP53 in Li-Fraumeni syndrome, and APC in familial adenomatous polyposis (Rahman 2014). All of these 114 CPG have derived from known genes, but none of them are fusion genes (Rahman 2014). Despite extensive research, known genetic factors can explain only a small percentage of familial cancer risk, implying that so-called low-hanging fruit of novel candidate genes remain to be discovered (Stadler, Schrader et al. 2014).

Recent rapid advances in RNA-seq make it possible to systematically discover fusion transcripts, and to use this technique for direct cancer diagnosis and prognosis (Mertens, Johansson et al. 2015). In the last several years, RNA-seq data have growing exponentially, and around 30,000 novel fusion transcripts and genes have been identified and accumulated by scientific and medical communities so far (Yoshihara, Wang et al. 2014, Mertens, Johansson et al. 2015).

The key challenge of this technique is how to fast and accurately map RNA-seq reads to the genomes. Although enormous progresses have been made, and more than 20 different software systems have been developed for the identification of fusion transcripts, none of these algorithms and software systems can achieve both fast speeds and high accuracies (Liu, Tsai et al. 2015).

US2016/078168 discloses compositions comprising probe pairs or primer pairs specific for detection and amplification of KANSARL fusion transcripts associated with cancer. However, it does not disclose present SEQ ID NO: 886551.

SUMMARY OF THE INVENTION

Previously, the applicant had disclosed a method of identifying fusion transcripts, whose content has been provided in U.S. Patent Application (Publication No. US20160078168 A1). In one aspect of the present disclosure, the applicant has used the method as disclosed above to analyze RNA-seq data from human cancer and other diseases, and has identified 886,543 novel fusion transcripts. A set of isolated, cloned recombinant or synthetic polynucleotides are herein provided. Each polynucleotide encodes a fusion transcript, the fusion transcript comprising a 5' portion from a first gene and a 3' portion from a second gene. The 5' portion from the first gene and the 3' portion from the second gene is connected at a junction; and the junction has a flanking sequence, comprising a sequence selected from the group of nucleotide sequences as set forth in SEQ ID NOs: 1-886,543 or from a complementary sequence thereof.

In a preferred embodiment, the present application provides a kit for detecting at least one KANSARL fusion transcript from a biological sample from a subject, comprising at least one of the following components: (a) at least one probe, wherein each of the at least one probe comprises a sequence that hybridizes specifically to a junction of the at least one KANSARL fusion transcript, said junction comprising a nucleotide sequence as set forth in SEQ ID NO: 886,551;(b) at least one pair of probes, wherein each of the at least one pair of probes comprises: a firstprobe comprising a sequence that hybridizes specifically to KANSL1; and a second probe comprising a sequence that hybridizes specifically to ARL17A, each of the at least one pair of probes comprises a pair of nucleotide sequences selected from one of SEQ ID NO: 886566 and SEQ ID NO: 886567; SEQ ID NO: 886568 and SEQ ID NO:886569; or(c) at least one pair of amplification primers, wherein each of the at least one pair of amplification primers are configured to specifically amplify the at least one KANSARL fusion transcript, each of the at least one pair of amplification primers comprises a pair of nucleotide sequences selected from one of SEQ ID NO: 886566 and SEQ ID NO: 886567; SEQ ID NO: 886568 and SEQ ID NO: 886569.

In some embodiments, the kit can further include compositions configured to extract RNA sample in the biological sample, and compositions configured to generate cDNA molecules from RNA sample in the biological sample.

The biological sample can be a cell line, buccal cells, adipose tissue, adrenal gland, ovary, appendix, bladder, bone marrow, cerebral cortex, colon, duodenum, endometrium, esophagus, fallopian tube, gall bladder, heart, kidney, liver, lung, lymph node, pancreas, placenta, prostate, rectum, salivary gland, skeletal muscle, skin, blood, small intestine, smooth muscle, spleen, stomach, testis, thyroid, and tonsil. The biological sample can be prepared in any methods. For example, the biological samples can be buccal cells prepared by buccal swabs, or can be a tissue sample prepared by biopsy, or can be a blood sample prepared by liquid biopsy. There are no limitations herein.

In embodiments of the kit comprising components as set forth in (a), the junction of the at least one KANSARL fusion transcript comprises a nucleotide sequence as set forth in SEQ ID NO: 886,551

Optionally, the components as set forth in (a) comprise a plurality of probes and a substrate, wherein the plurality of probes are immobilized on the substrate to thereby form a microarray. As such, the kit as set forth in (a) can be used to detect at least one KANSARL fusion transcript by microarray analysis, but the kit can also be used for analysis using other hybridization-based method.

In embodiments of the kit comprising components as set forth in (b), each of the at least one pair of probes comprises a pair of nucleotide sequences selected from one of SEQ ID NO: 886556 and SEQ ID NO: 886,567, SEQ ID NO: 886568 and SEQ ID NO: 886569,

Also disclosed are pairs of probe configured to detect the presence or absence of any of the KANSARL fusion transcript isoforms 1-6, among which, the probe pair SEQ ID NO: 886556 and SEQ ID NO: 886,567 is used for detection of isoform 1; the probe pair SEQ ID NO: 886566 and SEQ ID NO: 886567, and the probe pair SEQ ID NO: 886568 and SEQ ID NO: 886569, are used for isoform 2; the probe pair SEQ ID NO: 886560 and SEQ ID NO: 886561 for isoform 3; the probe pair SEQ ID NO: 886558 and SEQ ID NO: 886559 for isoform 4; the probe pair SEQ ID NO: 886564 and SEQ ID NO: 886565 for isoform 5; and the probe pair SEQ ID NO: 886562 and SEQ ID NO: 886563 for isoform 6, respectively.

These probe pairs are respectively used to detect the presence of any of the KANSARL fusion transcript isoforms by co-hybridization of the first probe and the second probe in a hybridization reaction, including in situ hybridization and Northern blot.

In some of the embodiments as described above, the first probe and the second probe respectively comprises a first moiety and a second moiety, configured to indicate co-hybridization of the first probe and the second probe in a hybridization reaction to thereby detect a presence of the at least one KANSARL fusion transcript. The first moiety and the second moiety can be fluorescence dyes, radioactive labels, or some other moiety capable of being conveniently recognized. The co-hybridization of the first probe and the second probe in a hybridization reaction refers to simultaneous detecting of the hybridization of the first probe and the second probe in one hybridization reaction. Examples include co-localization of the first probe and the second probe in an in situ hybridization assay, such as fluorescence in situ hybridization (FISH), and also include co-localization of the first probe and the second probe in a Northern blot analysis. There are no limitation herein.

In embodiments of the kit comprising components as set forth in (c), each of the at least one pair of amplification primers comprises a pair of nucleotide sequences selected from one of SEQ ID NO: 886556 and SEQ ID NO: 886,567, SEQ ID NO: 886568 and SEQ ID NO: 886569, Each of these pairs of amplification primers is configured to amplify the isoform of the KANSARL fusion transcript by PCR.

Among the primer pairs disclosed herein are, the primer pair SEQ ID NO: 886556 and SEQ ID NO: 886,567 is used for PCR amplification of isoform 1 (with an expected size of 379 bp for the PCR product); the primer pair SEQ ID NO: 886566 and SEQ ID NO: 886567, and the primer pair SEQ ID NO: 886568 and SEQ ID NO: 886569, are used for amplification of isoform 2 (with an expected size of 431 bp and 236 bp, respectively, for the PCR product); the primer pair SEQ ID NO: 886560 and SEQ ID NO: 886561 for amplification of isoform 3 (with an expected size of 149 bp for the PCR product); the primer pair SEQ ID NO: 886558 and SEQ ID NO: 886559 for amplification of isoform 4 (with an expected size of 385 bp for the PCR product); the primer pair SEQ ID NO: 886564 and SEQ ID NO: 886565 for amplification of isoform 5 (with an expected size of 304 bp for the PCR product); and the primer pair SEQ ID NO: 886562 and SEQ ID NO: 886563 for amplification of isoform 6 (with an expected size of 160 bp for the PCR product), respectively.

In some of the embodiments as disclosed above, the components of the kit as set forth in (c) can further comprise a DNA polymerase, configured to amplify the at least one KANSARL fusion transcript using the at least one pair of amplification primers. Optionally, the components of the kit as set forth in (c) can further include an instruction of how to perform the PCR reaction for amplification of the isoforms.

In a third aspect, the present disclosure provides a method for detecting presence or absence of at least one KANSARL fusion transcript in a biological sample from a subject utilizing the kit as described above. The method includes the steps of: (i) treating the biological sample to obtain a treated sample; (ii) contacting the treated sample with at least one components as set forth in (a), (b), or (c) of the kit for a reaction; and (iii) determining that the at least one KANSARL fusion transcript is present in the biological sample if the reaction generates a positive result, or that the at least one KANSARL fusion transcript is absent in the biological sample if otherwise.

In some embodiments of the method, the reaction in step (ii) can be a hybridization reaction. In some of the embodiments where the components as set forth in (b) are utilized, the positive result in step (iii) is co-localization of the first probe and the second probe in the hybridization reaction, and the hybridization reaction in step (ii) can be in situ hybridization (ISH) or Northern blot. In some of the embodiments where the components as set forth in (a) are utilized, the positive result in step (iii) is hybridization of the at least one probe with at least one polynucleotide in the treated sample. The hybridization reaction in step (ii) can be Southern blot, dot blot, or microarray, and the treated sample in step (i) can be a cDNA sample, and step (i) comprises the sub-steps of: isolating a RNA sample from the biological sample; and obtaining the cDNA sample from the RNA sample.

In some embodiments of the method, the reaction in step (ii) can be amplification reaction. Under such a case, the components as set forth in (c) are utilized, and the positive result in step (iii) is obtaining of at least one amplified polynucleotide of expected size. In preferred embodiments, step (iii) can further comprise verification of the at least one amplified polynucleotide by sequencing.

Also disclosed is a method for detecting the presence of KANSARL fusion gene from a genomic DNA sample of a subject. The method comprises: (i) contacting the treated sample with at least one primer pair for PCR amplification; and (ii) determining that the KANSARL fusion gene is present in the genomic DNA sample if the PCR amplification generates a positive result, or that the KANSARL fusion gene is absent in the genomic DNA sample if otherwise. Herein the positive result refers to the generation of a PCR product of expected size after PCR amplification. In some preferred embodiments, the PCR product can further undergo sequencing for verification. The genomic DNA sample can be prepared from a tissue sample, obtained from any method. For example, it can be prepared from buccal cells via buccal swabs.

BRIEF DESCRIPTION OF THE DRAWINGS

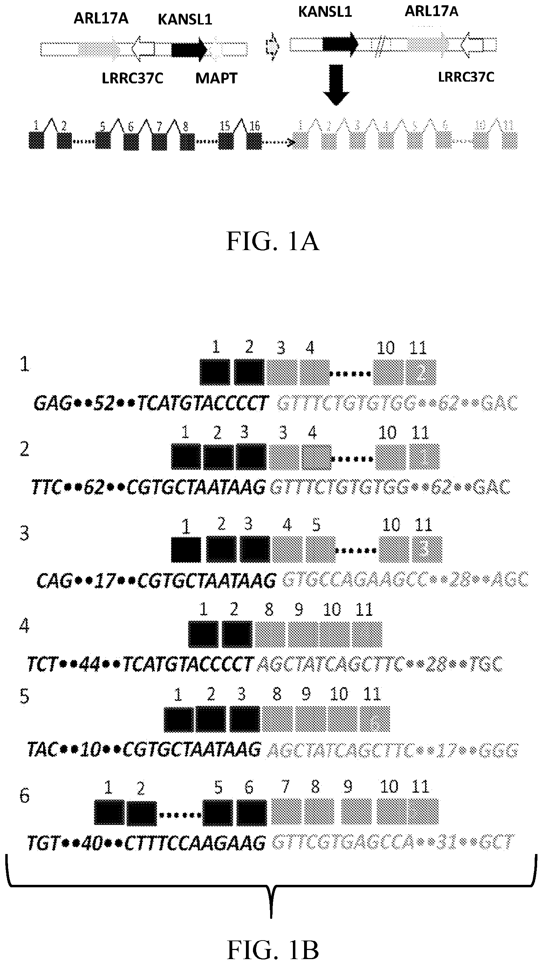

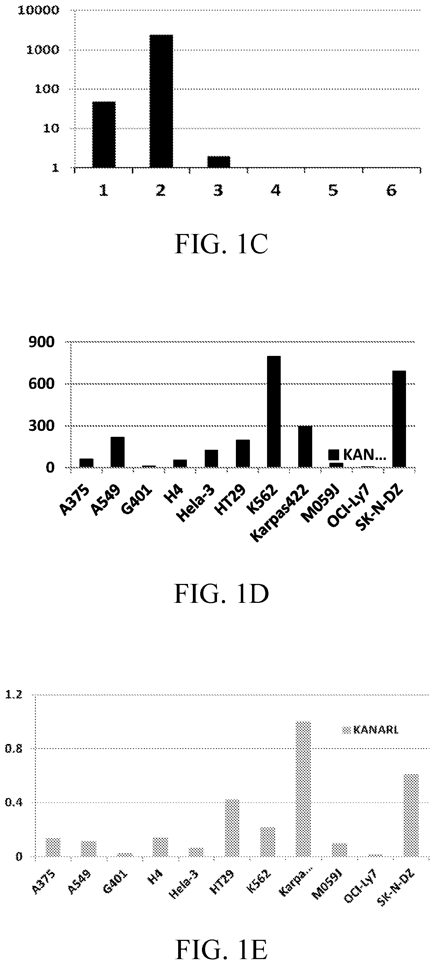

Figure 1 shows schematic diagrams of identification and characterization of KANSL1-ARL17A (KANSARL) fusion transcripts. a). Schematic diagrams of putative mechanisms via an inversion or a duplication to form a genomic structure of KANSL1 → ARL17A from a genomic structure of ARL17A → KANSL1. Solid black, gray and white arrows represent KANSL1, ARL17A and other genes indicated by letters and their orientations, respectively. Solid and grey squares represent KANSL1 and ARL17A exons, Vertical triangles are introns. Dashed lines show omitted exons and introns. Dashed lined horizontal arrow indicates unknown genomic sequences; b). Schematic diagrams show the six KANSARL isoforms identified and their junction sequences. The black and grey letters represent KANSL1 and ARL17A cDNA sequences; c) A graphic diagram shows the distribution of the raw counts of the six KANSARL fusion transcripts identified in the ECD39, where the numbers indicate the KANSARL isoforms; d). A graphic diagram shows that 11 cancer lines in the ECD39 have been identified to have KANSARL fusion transcripts. The black bars indicate raw counts of the total KANSARL fusion transcripts; e) A diagram shows distributions of normalized counts of KANSARL fusion transcripts observed in the 11 cancer lines. Y-axis represents the number of splice junctions per million reads (NSJPMR).

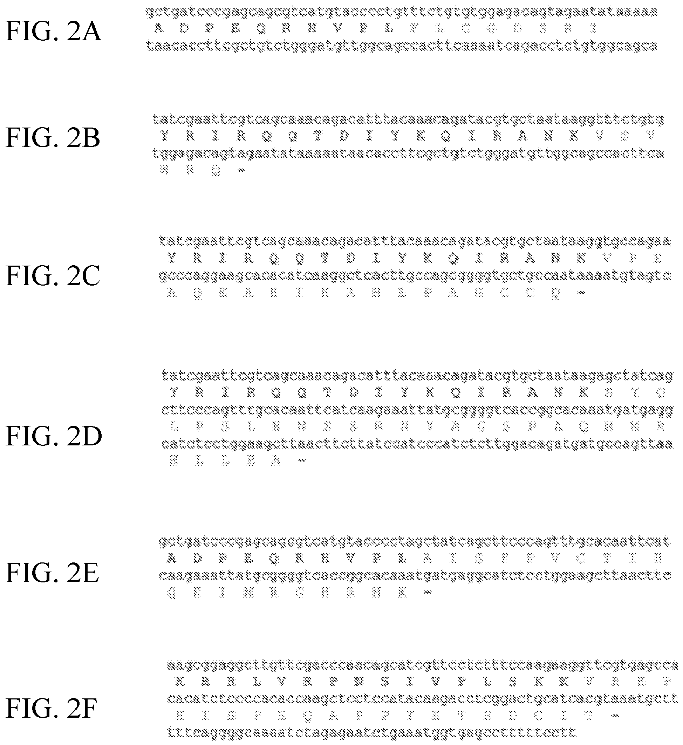

Figure 2 shows the KANSARL isoform RNA and protein sequences. a) KANSARL isoform 1; b) KANSARL isoform 2; c) KANSARL isoform 3; d) KANSARL isoform 4; e) KANSARL isoform 5; and f) KANSARL isoform 6; The black and underlined letters indicate peptide sequences from KANSL1 and ARL17A genes, respectively.

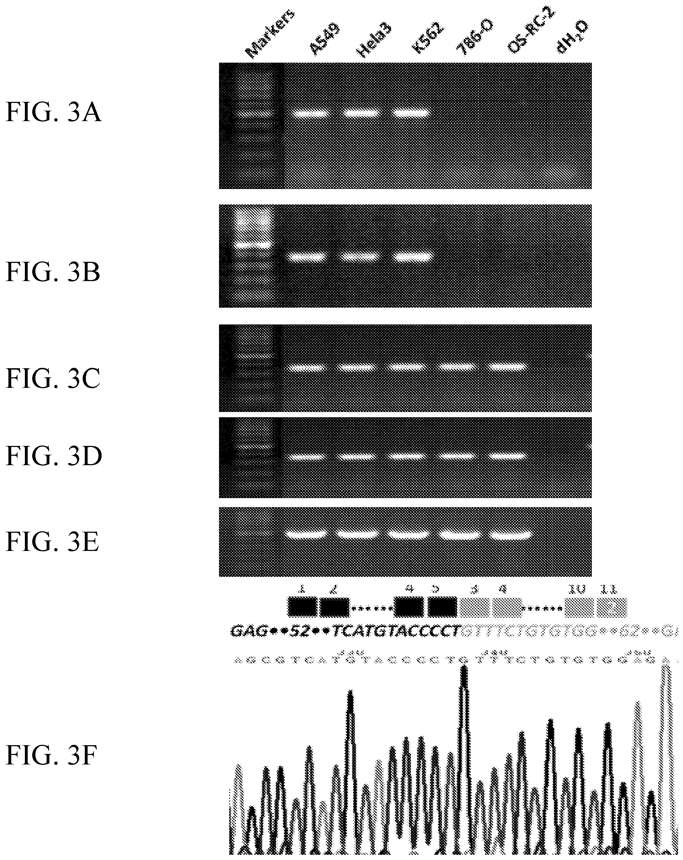

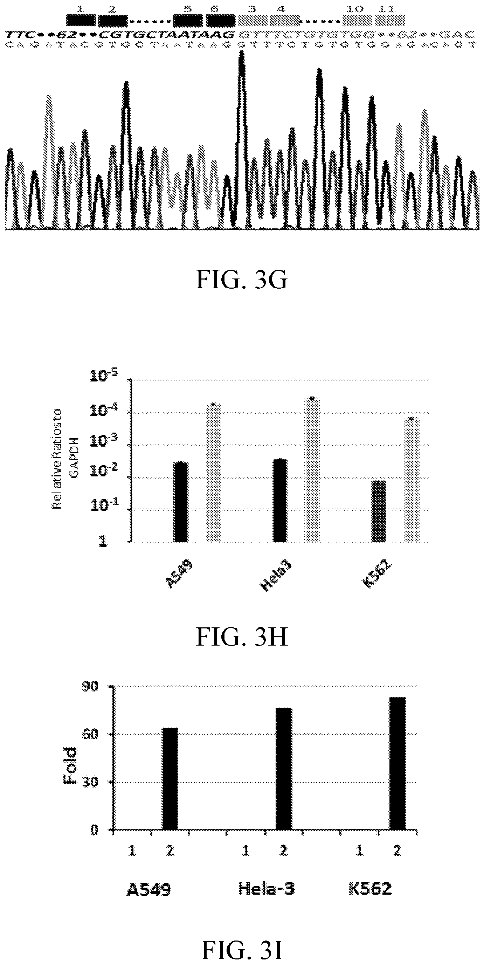

Figure 3 shows schematic procedure of validation of KANSARL isoform 1 and 2 in A549, Hela-3 and K562. a). RT-PCR amplification of KANSARL isoform 1 in A549, Hela-3, K562, 786-O and OS-RC-2; b). RT-PCR amplification of KANSARL isoform 2 in A549, Hela-3, K562, 786-O and OS-RC-2; c). RT-PCR amplification of KANSL1 in A549, Hela-3, K562, 786-O and OS-RC-2; d). RT-PCR amplification of ARL17A in A549, Hela-3, K562, 786-O and OS-RC-2; e). RT-PCR amplification of GAPDH in A549, Hela-3, K562, 786-O and OS-RC-2; f). Sequencing validation of KANSARL isoform 1 splice junctions; g). Sequencing validation of KANSARL isoform 2 splice junctions; h). A graphic diagram shows the relative expression levels of KANSARL isoform 1 and 2 in A549, Hela-3 and K562; and i). A graphic diagram shows the differences between KANSARL isoform 1 and 2 in A549, Hela-3 and K562. Y -axis indicates folds.

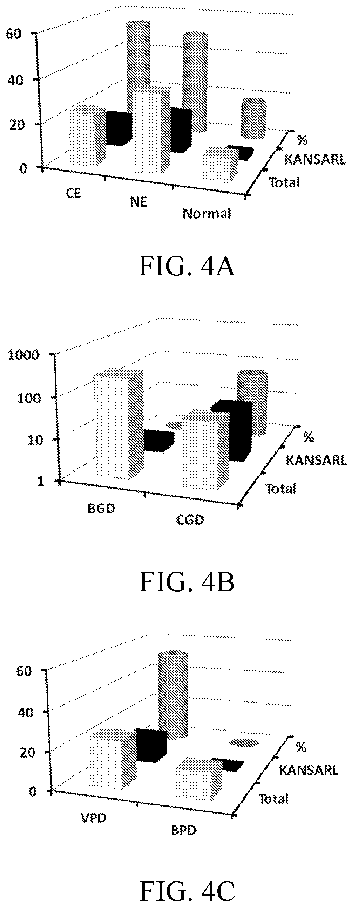

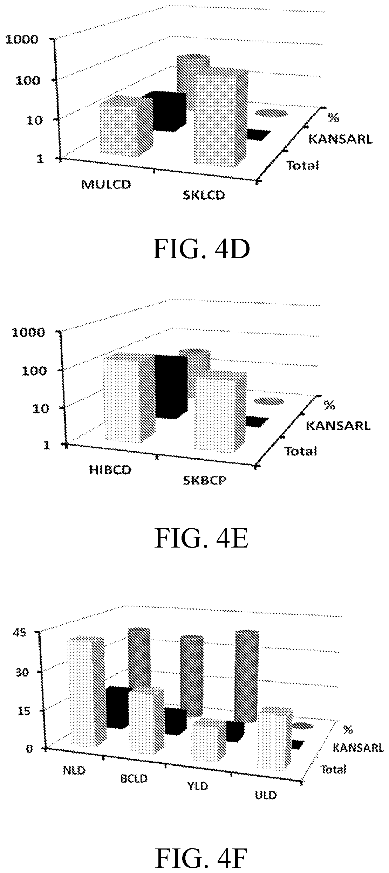

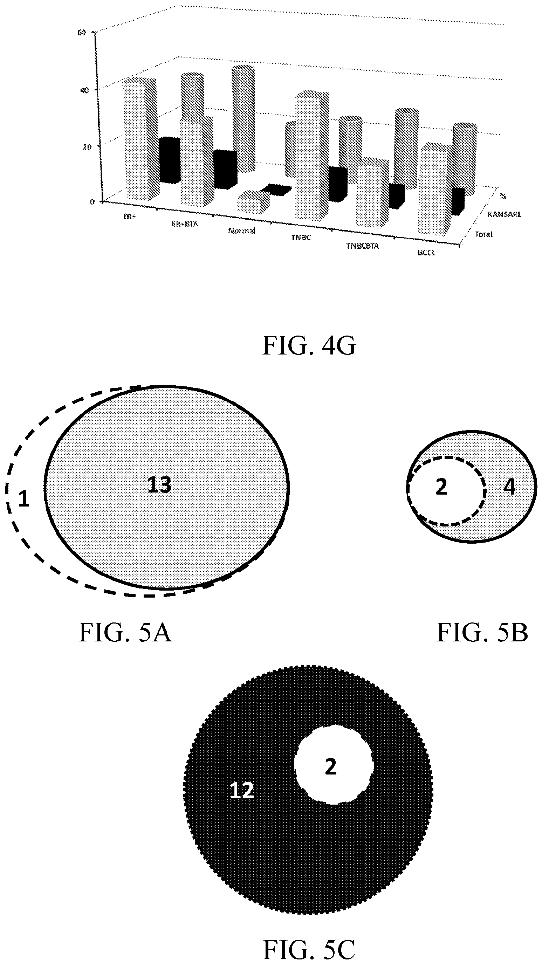

Figure 4 shows analyses of RNA-seq datasets from diverse types of cancer to illuminate that those KANSARL fusion transcripts are rarely found in cancer patients from Asia and Africa and are detected predominantly in cancer patients from North America. a). Analysis of KANSARL fusion transcripts in the CGD glioblastoma RNA-seq datasets. CE, NE and Normal represent contrast-enhancing regions (CE) of diffuse glioblastomas (GBM), nonenhancing regions (NE) of GBM and brain tissues of non-neoplastic persons as a normal control, respectively. b). Comparative analysis of KANSARL fusion transcripts between the BGD and CGD datasets. BGD and CGD represent 272 glioblastoma patients from Beijing Neurosurgical Institute and 27 glioblastoma patients of Columbia University Medical Center, respectively. c) Comparative analysis of KANSARL fusion transcripts between the VPD and BPD datasets. VPD and BPD are 25 prostate patient samples from Vancouver Prostate Centre and 14 prostate tumor samples from Beijing Genome Institute (BGI), respectively. d). Comparative analysis of KANSARL fusion transcripts between the MULCD and SLCD datasets. MULCD and SLCD represent 20 lung cancer patients from University of Michigan and 168 lung cancer samples from South Korean Genomic Medicine Institute. e). Comparative analysis of KANSARL fusion transcripts between the HIBCD and SKBPD datasets. HIBCD and SKBPD represent 163 breast cancer samples from Hudson Alpha Institute for Biotechnology and 78 breast cancer patients from South Korean, respectively. f). Comparative analysis of KANSARL fusion transcripts among the NLD, BCLD, YLD and ULD datasets. NLD, BCLD, YLD and ULD represent 41 sporadic forms Burkitt Lymphoma from National Cancer Institute, 13 cutaneous T cell lymphoma from Yale University, 23 diffuse large B-cell lymphoma from BC Cancer Agency, and 20 lymphoma samples from Uganda. Black and gray bars indicate total numbers of samples and numbers of samples having KANSARL fusion transcripts, respectively. Dark gray cylinders are percentages of KANSARL-positive samples in the datasets.

Figure 5 shows Venn diagrams of overlaps between KANSARL and TMPRSS2-ERG fusion transcripts. a). KANSARL+ tumors; b). KANSARL+ adjacent tissues; and c). KANSARL- tumors. Gray, white and black circles represent the KANSARL-positive, TMPRSS2-ERG and KANSARL-negative, respectively.

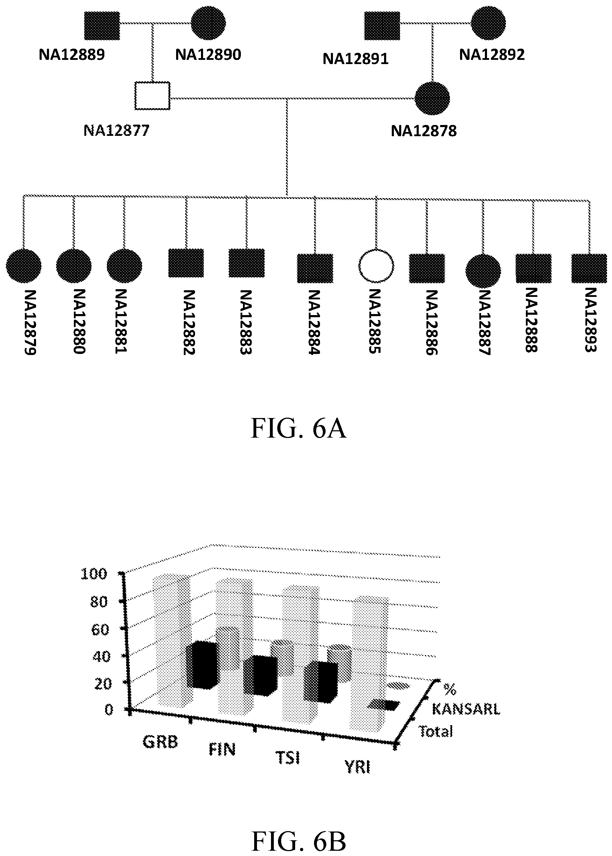

Figure 6 shows family inherence and population genetics of KANSARL fusion transcripts. a). Diagrams of KANSARL inheritance in the CEPH/Utah Pedigree 1463, which includes four grandparents, two parents and eleven children. Black and white squares represent KANSARL-positive and KANSARL-negative males while black and white squares indicate KANSARL-positive and KANSARL-negative females. The black lines are relationships among the family members. b). frequncies of KANSARL fusion transcripts in some populations of European and African ancestries. GBR is British from England and Scotland); FIN represents Finnish in Finland; TSI is Toscani in Italia; and YRI represents Yoruba in Ibadan, Nigeria.

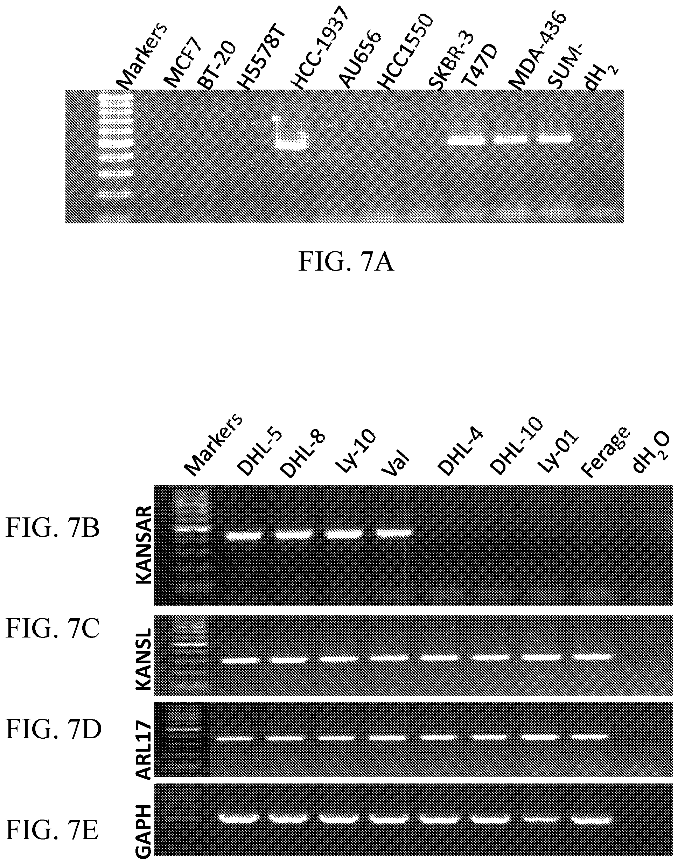

Figure 7 shows RNA-typing of KANSARL fusion transcripts in cancer cell lines. a). RT-PCR amplifications of breast cancer cell lines including MCF7, BT-20, H5578T, HCC1937, AU656,HCC1550, SKBR-3, T47D, MDA-436, SUM-159 and b). RT-PCR amplification of lymphomas cell lines including.DHL-5, DHL-8, Ly-10, Val, DLH-4, DHL-10, Ly-01, and Ferage c) KANSL1 gene; d) ARL17A; and e) GAPDH. All markers are 100bp DNA markers;

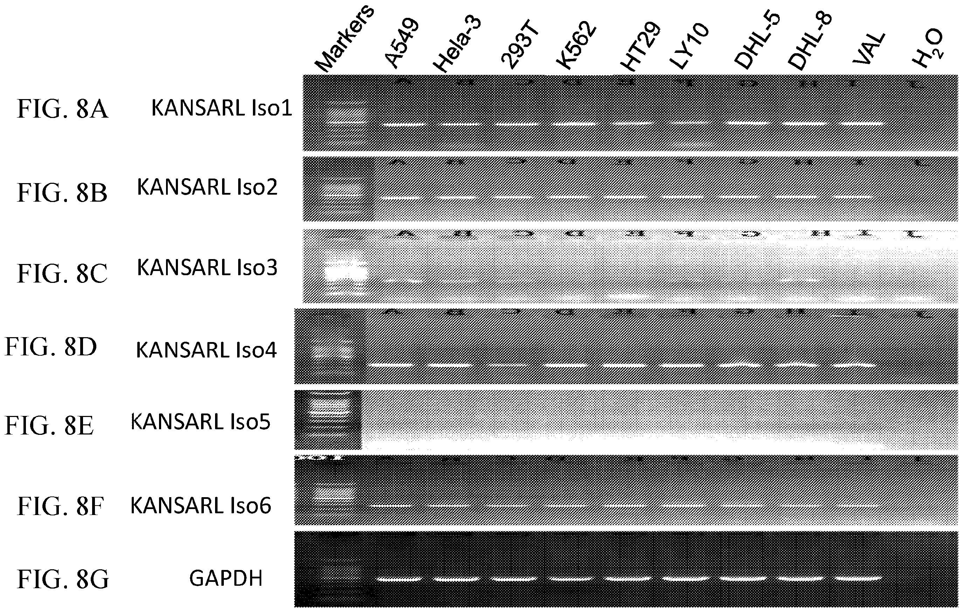

Figure 8 shows RT-PCR amplification of KANSARL isoforms. a). KANSARL isoform 1; b). KANSARL isoform 2; c). KANSARL isoform 3; d). KANSARL isoform 4; e). KANSARL isoform 5; f). KANSARL isoform 6; g). GAPDH was used as a control; DNA markers are 100 bp markers. Cell lines used for RT-PCR amplification include A549, Hela-3, 293T, K562, HT29, Ly-10 DHL-5, DHL-8, and Val.

BRIEF DESCRIPTION OF THE SEQUENCE LISTING

The instant disclosure includes a plurality of nucleotide sequences. Throughout the disclosure and the accompanying sequence listing, the WIPO Standard ST.25 (1998; hereinafter the "ST.25 Standard") is employed to identify nucleotides. The sequences of sequence ID 1 to sequence 886,543 are novel fusion transcripts. The sequences of sequence ID 886,544 to 886,549 are putative fusion polypeptides of KANSARL isoform 1, 2, 3, 4, 5 and 6. The sequences of sequence ID 886,550 to 886,555 are junction sequences of the putative fusion mRNA sequences of KANSARL isoform 1, 2, 3, 4, 5 and 6. The sequences from sequence ID 886,556 to sequence ID 886, 581 are primers used for RT-PCR and DNA amplifications.

DETAILED DESCRIPTION

Kinsella et al. have developed a method of ambiguously mapped RNA-seq reads to identify KANSL1-ARL17A fusion transcripts (Kinsella, Harismendy et al. 2011), which have been shown to have identical fusion junction with a cDNA clone of BC006271 (Strausberg et al. 2002). However, they are not verified experimentally. There is little information how this fusion transcript is related to cancer, which mutations cause fusion, which person has it, how it is inherited, and where it expressed.

KANSL1 and ARL17A genes are located at the chromosome 17q21.31. KANSL1 encodes an evolutionarily conserved nuclear protein, and is a subunit of both the MLL1 and NSL1 complexes, which are involved in histone acetylation and in catalyzing p53 Lys120 acetylation (Li, Wu et al. 2009). KANSL1 protein also ensures faithful segregation of the genome during mitosis (Meunier, Shvedunova et al. 2015). It has been found that there are two haplotypes, H1 and inverted H2 forms of which contain independently derived, partial duplications of the KANSL1 gene. These duplications have both recently risen to high allele frequencies (26% and 19%) in the populations of Europeans ancestry origin (Boettger, Handsaker et al. 2012). Some mutations have similar functions to the duplications, and both result in the Koolen-de Vries syndrome (KdVS) (OMIM #610443) characterised by developmental delay, intellectual disability, hypotonia, epilepsy, characteristic facial features, and congenital malformations in multiple organ systems (Koolen, Pfundt et al. 2015). ARL17A gene encodes a member of the ARF family of the Ras superfamily of small GTPases that are involved in multiple regulatory pathways altered in human carcinogenesis(Yendamuri, Trapasso et al. 2008).

Previously, we have observed that recently-gained human spliceosomal introns have a signature of identical 5' and 3' splice sites (Zhuo, Madden et al. 2007). Based on this finding, we have found that both 5' exonic sequences (E5) immediately upstream of introns and 3' intronic sequences (I3) were dynamically conserved, and appears rather reminiscent of self-splicing group II ribozymes and of constraints imposed by base pairing between intronic-binding sites (IBSs) and exonic-binding sites (EBSs). Therefore, we have proposed that both E5 and I3 sequences constitute splicing codes, which are deciphered by splicer proteins/RNAs via specific base-pairing(Zhuo D 2012). This splicing code model suggested that a yet-to-be characterized splicer proteins/RNA would decode identical sequences in all pre-mRNAs in conjugation with U snRNAs and spliceosomes, regardless whether the E5 and I3 sequences are in the one molecule or two different molecules. Using this splicing code model, we have developed a computation system to analyze RNA-seq datasets to study gene expression, to discover novel isoforms, and to identify fusion transcripts.

Based on our splicing code model, we have implemented a simple computation system to identify perfectly-identical fusion transcripts of two different traditional transcriptional units. In the previous application of U.S. Patent Application No. 14/792,613, filed July 07, 2015, we had used this splicingcode system to analyze RNA-seq datasets from cancer cell lines and cancer patients in ENCODE project and NCBI database and had identified 252,664 novel fusion transcripts. Since then, we have continued to analyze RNA-seq datasets from cancer, other disease and normal samples in the NCBI. After we removed the fusion transcripts identified previously, we have identified total 886,543 novel fusion transcripts of unique fusion junctions. The sequences of these fusion transcripts have been set forth in SEQ ID NOs.: 1-886,543.

To demonstrate the feasibilities and reliabilities of our approaches, we have selected KANSL1-ARL17A (KANSARL) fusion transcripts for systematical investigation. Existence and abundances of multiple KANSARL isoforms in a cell line rule out the possibilities that KANSARL fusion transcripts are *trans*-spliced products and therefore KANSL1 and ARL17A are adjacent. Figure la shows that a putative inversion or duplication of a normal genomic structure of ARL17A and KANSL1 genes at 17.q21.32 results in a inverted genomic structure of KANSL1 and ARL17A gene order (Figure la Right). Figure 1b has shown that six KANSARL fusion transcripts of unique splicing junctions have been identified in the ECD39 datasets, which are described in the previous patent application. From these six KANSARL isoforms, the KANSL1 gene has used three splice junctions of exons 2, 3 and 6, suggesting that 5' breakpoint occurs at least downstream of the exon 2 and it may be downstream of the exon 6 in some cell lines. ARL17A has returned exons 3, 4, 7 and 8, indicating that the 3' breakpoint occurs upstream of the ARL17A exon 3 (Figurelb). Sequence analysis has shown that the KANSARL isoform 2 has an identical fusion junction with a cDNA clone BC006271 (Strausberg, Feingold et al. 2002) and KANSL1-ARL17A fusion transcripts reported previously (Kinsella, Harismendy et al. 2011).

Figure 2 shows that the six KANSARL fusion transcripts encode 437, 483, 496, 505, 450 and 637 aa proteins, respectively, majorities of which are from KANSL1 sequences. Consequently, KANSARL fusion transcripts will retain only coiled coil domain and results in loss of WDR5 binding region, Zn finger, domain for KAT8 activity and PEHE, suggesting KANSARL fusion transcripts are similar to some KANSL1 mutations (Koolen, Pfundt et al. 2015).

To estimate gene expression levels of these six KANSARL isoforms in cancer, we have analyzed distribution of the copy numbers of the six fusion transcripts. Figure1c and Table 1 have shown distribution of raw counts of the six KANSARL fusion transcripts. The KANSARL isoform 2 is expressed at the highest levels among the six fusion transcripts and is 50 folds and 1216 folds higher than KANSARL isoform 1 and isoform 3.

Table 1: Distribution of KANSARL isoforms in the ECD39 dataset.

| KANSARL Isoforms | Counts | % of Total Expression | Folds |
|---|---|---|---|
| 1 | 48 | 1.93 | 50.69 |
| 2 | 2433 | 97.87 | 1 |
| 3 | 2 | 0.08 | 1216.5 |
| 4 | 1 | 0.04 | 2433 |
| 5 | 1 | 0.04 | 2433 |
| 6 | 1 | 0.04 | 2433 |

To study the KANSARL fusion transcript expression patterns in cancer cell lines, we have analyzed distribution of the total KANSARL fusion transcripts among the individual cell lines. Figure 1d and Table 2 have shown that the KANSARL fusion transcripts have been detected in 11 out of 39 cancer cell lines, which included A375, A549, G401, H4, Hela-3, HT29, K562, Karpas422, M059J, OCI-Ly7 and SK-N-DZ (Cautions should be taken for OCI-Ly7 since ENCSR001HHK dataset of Encode project is shown to be KANSARL-negative while ENCSR740DKM dataset is KANSARL-positive). As Table 2 shows, the KANSARL positive cells are from varieties of tissues and cell types as well as diversities of cancer types. Out of 11 cell lines of the positive KANSARL fusion transcripts, the genetic lineages are 6 Caucasian, one black (Hela-3) and 4 unknown genetic backgrounds. To rule out the effects of RNA-seq dataset sizes, we have normalized expression of the KANSARL fusion transcripts. Figure1e has shown that the highest expressed fusion transcripts have been found in Karapas-422 cancer cell line. A549, H4, HT29, A375, SK-N-SH, and K562 are among highly-expressed cancer cell lines.

Table 2: Basic information of KANSARL-positive cancer cell lines in the ECD39 dataset.

| Cell Lines | NSJPM | Tissues | Tumors | Sexes | Ages | Ethnic |
|---|---|---|---|---|---|---|
| A375 | 0.13 | Skin | malignant melanoma | Female | 54 | Caucasian |
| A549 | 0.11 | Lung | Carcinoma | Male | 58 | Caucasian |
| G401 | 0.03 | Kidney | rhabdoid tumor | Male | 0.25 | Caucasian |
| H4 | 0.14 | Brain | neuroglioma | Male | 37 | Caucasian |
| Hela-3 | 0.06 | cervix | adenocarcinoma | Female | 31 | Black |
| HT29 | 0.42 | colon | colorectal adenocarcinoma | Female | 44 | Caucasian |
| K562 | 0.22 | Bone Marrow | Leukemia | Female | 53 | Unknown |
| Karpas422 | 1.01 | B cells | non-Hodgkin's lymphoma | Female | 73 | Unknown |
| M059J | 0.09 | Brain | malignant glioblastoma | Male | 33 | Unknown |
| OCI-Ly7 | 0.02 | B cells | non-Hodgkin's lymphoma | Male | 48 | Unknown |
| SK-N-DZ | 0.61 | Brain | neuroblastoma | Female | 2 | Caucasian |

Since Figure 1d shows that A549, HeLa-3 and K562 express KANSARL fusion transcripts, we have then first sought to verify them at sequence levels. To this end, we have designated primers specific to all six KANSARL fusion transcripts to perform RT-PCR on total RNAs isolated from A549, HeLa-3 and K562 (Table 2) while cell lines 786-O and OS-RC-2 are used as negative controls. Figure 3a shows that amplification of A549, HeLa3 and K562 cDNAs by KANSARLIsoF1 (Seq ID NO.: 886,556) and KANSARLIsoR1 (Seq ID NO.: 888,557) generate expected 379 bp PCR fragments. Sequencing of the PCR fragments has confirmed that cDNAs have the splice junction generated by RNA-seq analysis (Figure 2f). Figure 2b shows that KANSARLF1 (Seq ID NO.: 886,566) and KANSARLR1 (Seq ID NO.: 886,567) are used to amplify A549, HeLa3 and K562 cDNAs to produce expected 431 bp fragments, which are confirmed by DNA sequencing to have the expected splice junction (Figure 3g). To check whether these KANSARL-positive cancer cell lines have intact KANSARL parental genes, KANSL1 and ARL17A, we have designated primers across breakpoints of both KANSL1 and ARL17 genes to perform RT-PCR amplification on these five cancer cell lines. Figure 2c & 2d have shown that A549, HeLa-3 and K562, similar to 786-O and OS-RC-2, have RT-PCR products detected, indicating that these cell lines have at least one copy of KANSL1 and one copy of ARL17A while PCR products generated by GAPDHF1 (Seq ID NO.: 886,570) and GAPDHR1(Seq ID NO.: 886,571) are used as a control (Figure 3e).

Table 3. The Primers Used to perform TR-PCR and Real-Time PCR

| Primer IDs | Primer Sequences | SEQ ID NO: | KANSARL Isoforms |
|---|---|---|---|
| KANARLIso1F1 | CAAGCCAAGCAGGTTGAGA | 886556 | |
| KANARLIso1RI | TCTCCACACAGAAACAGGGGTA | 886557 | 1 |
| | | | |
| KANARLIso4F1 | TTGTGCAAGCCAAGCAGGTT | 886558 | |
| KANARLIso4R1 | TGGGAAGCTGATAGCTAGGGGT | 886559 | 4 |
| | | | |
| KANARLIso3F1 | TCAGAATGGAAATGGGCTGCA | 886560 | |
| KANARLIso3R1 | TTCCTGGGCTTCTGGCACCTT | 886561 | 3 |
| | | | |
| KANARLIso6F1 | AGACGCAGGTCAGAATGGAAAT | 886562 | |
| KANARLIso6R1 | AAACTGGGAAGCTGATAGCTCT | 886563 | 6 |
| | | | |

(continued)

| Primer IDs | Primer Sequences | SEQ ID NO: | KANSARL Isoforms |
|---|---|---|---|
| KANARLIso5F1 | TGTCTTGGCAGACCACATTC | 886564 | |
| KANARLIso5R1 | GGAAAAAGGCTCACCATTTCA | 886565 | 5 |
| | | | |
| KANSARLF1 | GCCTTGAGAA AAGCTGCCAG | 886566 | |
| KANSARLR1 | aacatcccagacagcgaagg | 886567 | 2 |
| | | | |
| KANSARLF2 | GAGACGCAGGTCAGAATGGA | 886568 | |
| KANSARLR2 | Aaatgc tgc cac agaggtct | 886569 | 2 |
| | | | |
| GAPDHF1 | CAAGGTCATCCATGACAACTTTG | 886570 | |
| GAPDHR1 | GTCCACCACCCTGTTGCTGTAG | 886571 | |
| | | | |
| GAPDHqF1 | GCGACACCCACTCCTCCACCTTT | 886572 | |
| GAPDHqR1 | TGCTGTAGCCAAATTCGTTGTCATA | 886573 | |
| | | | |
| KANSARLgF1 | TGTGCAGCCTAAGCATGATCCT | 886574 | |
| KANSARLgR1 | GACACAGTGGCTCATGCCTGTAAT | 886575 | |

Figure 3a & 3b demonstrate that A549, Hela-3 and K562 express both KANSARL isoform 1 and 2 and Table 1 shows that the counts of KANSARL isoform 2 reads is 50-fold higher than those of KANSARL isoform 1, suggesting that KANSARL isoform 2 is expressed at much higher level than the KANSARL isoform 1. To establish a relationship between KANSARL expression levels and counts of RNA-seq reads crossing splice junctions, we have performed real-time PCR of A549, Hela-3 and K562 on KANARL isoform 1 by KANSARLIsoFl (Seq ID NO.: 886,556) and KANSARLIsoR1 (Seq ID NO.: 886,557) and on KANARL isoform 2 by KANSARLF2 (Seq ID NO.: 886,568) and KANSARLR2 (Seq ID NO.: 886,569) while products amplified by GAPDHqF1 (Seq ID NO.: 886,572) and GAPDHqR1 (Seq ID NO.: 886,573) is used as reference control. Figure 3h shows relative expression levels of KANSARL isoform 1 (grey bars) and 2 (black bars) in A549, Hela-3 and K562.

Table 4 shows that KANSARL isoform 2 are expressed at 0.35%, 0.28% and 1.28% of the GAPHD expression in A549, Hela-3 and K562, respectively while KANSARL isoform 1 are expressed only at 0.0056%, 0.0037% and 0.015% of the GAPHD expression in A549, Hela-3 and K562, respectively. Figure 3i and Table 4 show that KANSARL isoform 2 (black bars) are expressed at average 73 fold higher than KANSARL isoform 1 (gray bars), which ranged from 63.7 folds in A549 to 82.9 folds in K562. These qPCR differences between two KANSARL isoforms are generally consistent with that obtained from RNA-seq data analysis (Figure lc&d). K562 expresses KANSARL isoform 2 at 1.2% of GAPDH gene expression levels; while A549 and Hela-3 express this isoform at 0.3% of GAPDH ones (Figure 3h). The former level is about 4 fold of the latter, which are also consistent with data obtained from RNA-seq data analysis (Figure 1e). Further study is required to confirm whether four folds of the qPCR differences between A549 and Hela-3/K562 are genotype differences between KANSARL[+]/KANSARL[+] and KANSARL[+]/KANSARL[-] or gene expression differences among different cancer types.

Table 4 Real-time PCR Quantifications of KANSARL isoform 1 and 2 in A549, Hela-3 and K562.

| | | Ratios of KANSARL soforms and GAPHD Expression Levels | | | | | |
|---|---|---|---|---|---|---|---|
| Cell Lines | Gene / Isoforms | Rep 1 | Rep 2 | Rep 3 | Average | SD | Folds |
| A549 | GAPHD | 1 | 1 | 1 | 1 | 0 | |
| | KANSARL Iso2 | 0.0035 | 0.0037 | 0.0036 | 0.0036 | 0.000087 | 63.7 |
| | KANSARL Iso1 | 5.69E-05 | 5.5E-05 | 5.7E-05 | 5.6E-05 | 1.12E-06 | |

(continued)

| Ratios of KANSARL soforms and GAPHD Expression Levels | | | | | | |
|---|---|---|---|---|---|---|
| Cell Lines | Gene / Isoforms | Rep 1 | Rep 2 | Rep 3 | Average | SD | Folds |
| | | | | | | | |
| Hela-3 | GAPHD | 1 | 1 | 1 | 1 | 0 | |
| | KANSARL Iso2 | 0.00288 | 0.00288 | 0.00278 | 0.0028 | 5.66E-05 | 76.3 |
| | KANSARL Iso1 | 3.58E-05 | 3.8E-05 | 3.8E-05 | 3.7E-05 | 1.36E-06 | |
| | | | | | | | |
| K562 | GAPHD | 1 | 1 | 1 | 1 | 0 | |
| | KANSARL Iso2 | 0.0129 | 0.0128 | 0.0128 | 0.0128 | 5.132E-05 | 82.9 |
| | KANSARL Iso1 | 0.0002 | 0.0002 | 0.0002 | 0.00015 | 1.07E-06 | |

As Table 2 shows that KANSARL fusion transcripts are expressed in diverse cancer types, this has prompted us to analyze RNA-seq data from varieties of cancer types to identify and characterize KANSARL gene expression among diverse cancer RNA-seq datasets. To investigate whether KANSARL fusion transcripts are expressed in brain cancer and tissues, we have downloaded and analyzed the glioblastoma RNA-seq dataset of Columbia University Medical Center (designated as CGD), which has total of 94 samples included 39 contrast-enhancing regions (CE) of diffuse glioblastomas (GBM), 36 nonenhancing regions of GBM (NE) and 19 non-neoplastic brain tissues (Normal) from 17 samples (Gill, Pisapia et al. 2014). The CGD has total 27 patients and both CE and NE datasets have 24 patients, respectively, 21 of which are overlapped.

Figure3a and Table 5 show that KANSARL fusion transcripts have been found in 13 CE patients and 11 NE patients. Together, 14 (51.9%) of the 27 GBM patients have been found to have fusion transcripts. In contrast, KANSARL fusion transcripts have been detected only in 2 (or 11.7%) of 17 non-neoplastic brain tissues. The KANSARL-positive glioblastomas patients are 30% higher than the non-neoplastic persons (Figure 4a). The difference is shown to be statistically significant (Z=2.03, p<0.04), demonstrating that KANSARL fusion transcripts are associated with diffuse glioblastomas. In contrast, the difference in numbers of KANSARL-positive NC and NE samples is statistically insignificant (Z=0.577, p>0.8), suggesting that KANSARL genotypes of the NE samples are similar to those of the NC samples.

Table 5 Statistical analysis of number differences of KANSARL+ samples between glioblastomas CE and NE samples

| Types | # of Samples | # of KANSARL+ | % ofKANSARL+ | Z Scores | probabilities |
|---|---|---|---|---|---|
| CE | 24 | 13 | 54.17 | 0.577 | 0.5637 |
| NE | 24 | 11 | 45.83 | | |

Table 6 Comparison of number differences of KANSARL+ samples between glioblastomas and non-neoplastic samples

| Types | # of Samples | # ofKANSARL+ | % of KANSARL+ | Z Scores | probabilities |
|---|---|---|---|---|---|
| Total | 27 | 14 | 51.85 | 2.029 | 0.042 |
| Normal | 17 | 2 | 11.76 | | |

Since we have shown that KANSARL fusion transcripts are associated with diffuse glioblastomas, to characterize that KANSARL fusion transcripts in other glioblastoma datasets, we have performed comparative analysis of the glioblastoma dataset deposited by Beijing Neurosurgical Institute (designated as BGD), which have 272 gliomas of different clinic prognosis stages (Bao, Chen et al. 2014). Surprisingly, only two KANSARL-positive samples have been detected out of 272 BGD glioblastoma (Figure 4b). Only less than 1% of BGD glioblastoma is KANSARL-positive and is 52 times lower than that in the CGD dataset. Table 7 shows that the difference between BGD and CGD is statistically significant (Z=11.26, p<0.0005), suggesting that the BGD's KANSARL genotypes are divergent from those of CGD. Larger numbers of high-quality RNA-seq reads per sample in the BGD's dataset rule out the possibilities that the RNA-seq datasets are responsible for the difference between the two datasets (Gill, Pisapia et al. 2014).

Table 7 Comparison of number differences of KANSARL+ samples between BGD and CGD samples

| Types | # of Samples | # of KANSARL+ | % of KANSARL+ | Z Scores | Probabilities |
|---|---|---|---|---|---|
| BGD | 272 | 2 | 0.74 | 11.26 | < 0.00001 |
| CGD | 27 | 14 | 51.85 | | |

The dramatic differences of KANSARL fusion transcripts between the CGD and BGD have raised the possibility that KANSAR fusion transcripts are associated with the cancer patients of European ancestry origins, but absent in cancer patients of Asian ancestry. To study this possibility, we have systematically performed comparative analyses of RNA-seq datasets of prostate cancer, breast cancer, lung cancer and lymphomas around the world. Prostate cancer is the most common nonskin cancer and the second leading cause of cancer-related death in men in the United States. We have downloaded and performed analysis of the prostate cancer dataset from Vancouver Prostate Centre (designated as VPD), which contains 25 high-risk primary prostate tumors and five matched adjacent benign prostate tissues (Wyatt, Mo et al. 2014), and BGI prostate cancer dataset (BPD), which contain 14 pairs of prostate cancer and normal samples (Ren, Peng et al. 2012). We have detected KANSARL fusion transcripts in 13 (52%) out of the 25 VPD prostate samples (Figure4c) and 4 out of 5 adjacent benign prostate tissues.. KANSARL isoform 1, 2, and 3 have been detected in the VPD samples and have very similar patterns to those observed in ECD39 (Figure 1c). In contrast, we have found no single copy of KANSARL fusion transcript in the BPD prostate tumors and their matched normal samples (Figure 4c). Table 8 shows that the difference between VPD and BPD is statistically significant (z= 3.118; p < 0.05). It is well known that TMPRSS2-ERG is one of the most frequent fusion genes in prostate tumors (Wyatt, Mo et al. 2014). To investigate relationship between KANSARL and TMPRSS2-ERG fusion transcripts, we have performed analysis of TMPRSS2-ERG fusion transcripts and have detected 15 out 25 prostate tumors to have TMPRSS2-ERG fusion transcripts. Even more surprisingly, 13 out of 15 TMPRSS2-ERG-positive prostate tumors are KANSARL-positive or all of 13 KANSARL-positive prostate tumors are shown to have TMPRSS2-ERG fusion transcripts (Figure 5a). In contrast, only two TMPRSS2-ERG-positive prostate tumors are detected in 12 KANSARL-negative prostate tumors (Figure 5b).Table 8 shows that the differences of TMPRSS2-ERG fusion transcripts between KANARL-positive and KANSARL-negative tumors is significant (z=4.25, p<0.0005), suggesting that KANSARL fusion transcripts are closely associated with TMPRSS2-ERG and may play roles in generating TMPRSS2-ERG in prostate tumors. On the other hand, two samples out of 5 adjacent benign prostate tissues have been shown to have both TMPRSS2-ERG and KANSARL fusion transcripts (Figure 5c), suggesting that prostate tumor cells are present the adjacent benign tissues. In contrast, only one BPD's patient has been found to have TMPRSS2-ERG fusion transcripts.

Table 8 Comparison of number differences of KANSARL+ samples between VPD and BPD samples

| Types | # of Samples | # ofKANSARL+ | % ofKANSARL+ | Z Scores | probabilities |
|---|---|---|---|---|---|
| VPD | 25 | 13 | 52 | 3.118 | 0.002 |
| BPD | 14 | 0 | 0 | | |

Table 9: Overlaps between KANSAL and TMPRSS2-ERG fusion transcripts in the VPD samples

| Sample Types | # of Samples | # of TMPRSS2-ERG+ | % of TMPRSS2-ERG+ | Z Scores | probabilities |
|---|---|---|---|---|---|
| KANSARL+ | 13 | 13 | 100 | 4.25 | 2.10E-05 |
| KANSARL- | 12 | 2 | 16.67 | | |

To investigate whether KANSARL fusion transcripts are associated with other fusion transcripts, we have investigated differentially expressed fusion transcripts in both VPD prostate and CGD glioblastomas. To count fusion transcripts as a differentially-expressed fusion transcripts in cancer, fusion transcripts must have ≥75% of ≥5 samples in one group. Supplementary Table 9 shows that KANSARL-positive prostate cancer patients 26 differentially-expressed fusion transcripts, 81% of them are read-through (epigenetic) fusion transcripts while KANSARL-negative patients have 16 differentially-expressed fusion transcripts, 69% of which are read-through fusion transcripts. On the other hand, KANSARL-positive glioblastomas patients have 20 differentially-expressed fusion transcripts, 95% of which are read through while KANSARL-negative glioblastomas patients have only 6 differentially-expressed fusion transcripts, all of which are breakthroughs (Table 10). Data analysis shows that there are no overlapped fusion transcripts between prostate cancer and glioblastomas patients, suggesting these fusion transcripts are tissue-specific and caner-specific.

Table 10 Comparison of differentially-expressed fusion transcripts in KANSARL-positive and KANSARL-negative patients.

| a | Prostate Cancer | | | |
|---|---|---|---|---|
| | KANSARL-positive | | KANSARL-negative | |
| | Counts | % | Counts | % |
| Genetic | 5 | *19.23* | 5 | 31.25 |
| Epigenetic | 21 | 80.77 | 11 | 68.75 |
| Total | 26 | | 16 | |
| | | | | |
| | | | | |
| b | | Glioblastomas | | |
| | KANSARL-positive | | KANSARL-negative | |
| | Counts | % | Counts | % |
| Genetic | 1 | 5 | 0 | 0 |
| Epigenetic | 19 | 95 | 6 | 100 |
| Total | 20 | | 6 | |
| | | | | |

Lung cancer is the leading cause of cancer deaths in the World, especially in Asia. To investigate the expression of KANSARL fusion transcripts, we have analyzed the Korean Lung Cancer RNA-seq dataset (designated as SKLCD), which has 168 lung cancer samples (Ju, Lee et al. 2012) and Michigan of University Lung Cancer Dataset (designated as MULCD), which contains 20 lung tissue samples (Balbin, Malik et al. 2015). We have found that eight (40%) out of 20 MULCD samples have KANSARL fusion transcripts (Figure 4d). Even though SKLCD data are more than five folds larger than the MULCD ones, no single copy of KANSARL fusion transcripts have been detected in 168 SKLCD samples (Figure 4d). Table 11 shows that the differences of KANSARL fusion transcripts between MULCD and SKLCD is significant (z=8.38, p<0.0005), suggesting that KANSARL fusion transcripts are associated with MULCD lung cancer patients.

Table 11 Comparison of number differences of KANSARL+ samples between MULCD and SKLCD samples

| Types | # of Samples | # of KANSARL+ | % ofKANSARL+ | Z Scores | probabilities |
|---|---|---|---|---|---|
| MULCD | 20 | 8 | 40 | 8.3777483 | < 0.00001 |
| SKLCD | 168 | 0 | 0 | | |

Breast Cancer is the most common incident form of cancer in women around the world and about 1 in 8 (12%) women in the US will develop invasive breast cancer during their lifetime. To investigate whether KANSARL fusion transcripts are expressed in breast cancer, we have performed analyses on the breast cancer dataset from USA Hudson Alpha Institute for Biotechnology (designated as HIBCD), which consists of 28 breast cancer cell lines, 42 ER+ breast cancer primary tumors, 30 uninvolved breast tissues adjacent to ER+ primary tumors, 42 triple negative breast cancer (TNBC) primary tumors, 21 uninvolved breast tissues adjacent to TNBC primary tumors and 5 normal breast tissues(Varley, Gertz et al. 2014), and breast cancer samples from South Korean (designated as SKBCP), which have samples from 22 HRM (high-risk for distant metastasis) and 56 LRM (low-risk for distant metastasis) breast cancer patients (PRJEB9083 2015). Figure 4e shows that 50 (or about 30%) HIBCD breast samples have been found to have KANSARL fusion transcripts while no SKBCP patients have been observed to have KANSARL fusion transcripts. Table 12 shows that the difference between HIBCD and SKBCP has been shown by $\chi$2-test to be statistically significant (p$\leq$0.001), suggesting that breast cancer patients from South Korea have no KANSARL fusion transcripts.

Table 12 Comparison of number differences of KANSARL+ samples between HIBCD and SKBCP samples

| Types | # of Samples | # ofKANSARL+ | % ofKANSARL+ | Z Scores | probabilities |
|---|---|---|---|---|---|
| HIBCD | 163 | 49 | 30.06 | 5.43 | < 0.00001 |
| SKBCP | 78 | 0 | 0 | | |

Since HIBCD have multiple breast cancer types, we have performed further data analysis of the HIBCD breast samples. Figure 4g and Table 13 shows that normal tissues, breast cancer cell lines, TNBC primary tumors and uninvolved breast tissues adjacent to TNBC primary tumors have 23.8% to 28.5% of KANSARL-positive samples while ER+ breast cancer primary tumors and uninvolved breast tissues adjacent to ER+ primary tumor are 35.7% and 40%. KANSARL-positive percentages of The TNBC samples are much closer to the normal one, which are shown to have no statistical differences. On the other hand, the KANSARL-positive ratios in the ER+ samples are 15% higher than the normal one, suggesting that KANSARL fusion transcripts have much bigger impacts on ER+ breast cancer than TNBC breast cancer.

Table 13 Comparison of number differences of KANSARL+ samples among different subtypes of breast cancers in HIBCD samples

| Types | # of Samples | # of KANSARL+ | % of KANSARL+ | Z Scores | probabilities |
|---|---|---|---|---|---|
| ER+ | 42 | 15 | 35.71 | -0.386 | 0.699 |
| ER+BTA | 30 | 12 | 40 | 0.882 | 0.378 |
| Normal | 5 | 1 | 20 | -0.188 | 0.852 |
| TNBC | 42 | 10 | 23.81 | -0.416 | 0.498 |
| TNBCBTA | 21 | 6 | 28.57 | 0.280 | 0.779 |
| BCCL | 28 | 7 | 25 | | |

To investigate whether the KANSARL fusion transcripts are expressed in cancer samples from the African population, we have analyzed the Uganda lymphomas dataset (designated as ULD), which contains 20 lymphoma samples (Abate, Ambrosio et al. 2015). We have performed analyses of multiple lymphoma RNA-seq datasets including NCI lymphoma dataset (designated as NLD), which has 28 sporadic form Burkitt Lymphoma (BL) patient biopsy samples and 13 BL cell lines(Schmitz, Young et al. 2012), Yale University T-cell lymphoma dataset (designated as YLD), which has 13 cutaneous T cell lymphoma and BC Cancer Agency lymphoma data (designated as BLD), in which 23 RNA-seq data of diffuse large B-cell lymphoma have been identified (Morin, Mungall et al. 2013). Even though lymphoma subtypes and the sample sizes are different, we have found that have 34% to 38% of NLD, YLD and BLD samples have KANSARL fusion transcripts (Figure 4f). On the other hand, no single copy of KANSARL fusion transcripts have been detected in 20 ULD lymphoma samples (Figure 4f). Table 14 shows that the differences of KANSARL-positive samples between Uganda and North America are statistically significant (Z$\geq$3.0; p$\leq$ 0.0026) and suggested that Uganda lymphomas are not associated with KANSARL fusion transcripts.

Table 14 Comparison of number differences of KANSARL+ samples among the NLD, BCLD, YLD and ULD samples

| Types | # of Samples | # of KANSARL+ | % of KANSARL+ | Z Scores | probabilities |
|---|---|---|---|---|---|
| NLD | 41 | 15 | 36.59 | 3.11 | 0.002 |
| BCLD | 23 | 8 | 34.78 | 3.23 | 0.001 |
| YLD | 13 | 5 | 38.46 | 3.01 | 0.003 |
| ULD | 20 | 0 | 0 | | |

As shown in Figure 4, samples of diverse types of cancer from North America (USA and Canada) have been found to have highly recurrent KANSARL fusion transcripts, which ranged from 30% in breast cancer to 52% in prostate tumors. In contrast, KANSARL fusion transcripts have been detected in two glioblastoma samples from China and Hela-3 cancer cell line, ethnicity of which is black. No KANSARL fusion transcripts have been found in the rest of the cancer samples from South Korea, China and Uganda. Based on localities of health services, we can conclude that KANSARL fusion transcripts have been rarely found in the cancer samples from Asian and African ancestry origins and are specifically associated with cancer samples of European ancestry origins.

Presence of KANSARL fusion transcripts in normal and adjacent tissues raised the possibility that KANSARL fusion transcripts are an inherited germline fusion gene. To test this possibility, we have performed RNA-seq data analysis of the lymphoblastoid cell lines of families from the CEU population (CEPH/Utah Pedigree 1463, Utah residents with ancestry from northern and western Europe), which has a 17-individual, three-generation family (Li, Battle et al. 2014). Table 15 shows that KANSARL fusion transcripts have been detected in 15 of 17 family members as indicated by black squares and circles (Figure 6a). Only the father (NA12877) and daughter (NA12885) are not KANSARL carriers. Based on these data, if we can assume that the father and mother is KANSARL⁻/KANARL⁻, and KANSARL⁺/KANARL⁺, their sons and daughters would have KANSARL⁺/KANARL⁻ except for one daughter. The daughter (NA12885) is an outlier, which may have the mutated gene or may be promiscuous. However, based on the RNA-seq data, the more reasonable explanation is that the father (NA12877) may be mixed up with one of his sons during experiments and have a genotype of KANSAR⁺/KANARL⁻. Consequently, their sons and daughters would have one quarter of KANSARL⁻/KANARL⁻ and are well fit with what is predicted by Mendel's law.

Table 15 Distribution of KANSARL fusion transcripts in the CEPH/Utah Pedigree 1463

| Individual ID | Run ID | MB | KANSARL+ |
|---|---|---|---|
| NA12877 | SRR1258217 | 4670 | 0 |
| NA12878 | SRR1258218 | 3709 | 10 |
| NA12879 | SRR1258219 | 4759 | 11 |
| NA12880 | SRR1258220 | 4523 | 3 |
| NA12881 | SRR1258221 | 3548 | 7 |
| NA12887 | SRR1258222 | 3900 | 5 |
| NA12888 | SRR1258223 | 3141 | 2 |
| NA12892 | SRR1258224 | 3509 | 7 |
| NA12893 | SRR1258225 | 3529 | 8 |
| NA12882 | SRR1258226 | 3801 | 10 |
| . NA12883 | SRR1258227 | 2644 | 3 |
| NA12884 | SRR1258228 | 3086 | 4 |
| NA12885 | SRR1258229 | 4242 | 0 |
| NA12886 | SRR1258230 | 3485 | 11 |
| NA12889 | SRR1258231 | 3313 | 15 |
| NA12890 | SRR1258232 | 3145 | 1 |
| NA12891 | SRR1258233 | 3189 | 5 |

Figure 4 shows that KANSARL fusion transcripts are rarely detected in cancer samples from Asia and Africa, but are observed in 30-52% of tumor samples from North America and Figure 6a shows that KANSARL fusion transcripts are an inherited germline fusion gene. To estimate the percentages of general populations, we have downloaded and analyzed RNA-seq data analysis of the lymphoblastoid cell lines of the 1000 Genome Project (Genomes Project, Auton et al. 2015). Table 16 has shown that no single copy of KANSARL fusion transcripts has been detected in the Nigeria YRI (Yoruba in Ibadan) populations and that KANSARL fusion transcripts have been found in 33.7% GBR (British from England and Scotland), 26.3% FIN (Finnish in Finland) and 26.9% TSI (Toscani in Italia) populations, respectively (Figure 6b). Table 16 shows that the differences of KANSARL frequencies among the GBR, FIN and TSI populations are not statistically significant (Z≤1.11, p>0.27), suggesting these differences may be caused by sampling errors. On the other hand, their difference with the YRI KANSARL frequencies is statistically significant (Z≥5.2; p< 0.00001), confirming the previous observation that KANSARL fusion transcripts rarely exist in the tumor samples from African ancestry.

Table 16 Comparison of KANSARL frequency differences of GBR, FIN, TSI and YRI populations

| Sample IDs | # of Samples | # ofKANSARL | % of KANSARL | Z Scores | probabilities |
|---|---|---|---|---|---|
| GRB | 95 | 32 | 33.68 | 6.024 | < 0.00001 |

(continued)

| Sample IDs | # of Samples | # ofKANSARL | % of KANSARL | Z Scores | probabilities |
|---|---|---|---|---|---|
| FIN | 95 | 25 | 26.32 | 5.206 | < 0.00001 |
| TSI | 93 | 25 | 26.88 | 5.266 | < 0.00001 |
| YRI | 89 | 0 | 0.00 | | |

As shown above, KANSARL fusion transcripts seem to be expressed in many human tissues and organs. To systematically understand the patterns of KANSARL gene expression in human bodies, we have downloaded and analyzed RNA-seq datasets from Science for Life Laboratory, Sweden (designated as SSTD), which originated from tissue samples of 127 human individuals representing 32 different tissues(Uhlen, Fagerberg et al. 2015). Table 17 shows that KANSARL fusion transcripts have been detected in 28 of 32 tissues analyzed. Only bone marrow, kidney, stomach and smooth muscle have not been found to have KANSARL fusion transcripts. Since G401 and K562 originated from Kidney and bone marrow, respectively, our data suggest that KANSARL transcripts are expressed in the most human tissues if they are not ubiquitously expressed in the human tissues and organs and may be similar to the KANSL1 gene expression patterns.

| Table 17 - Distribution of KARSARL fusion transcripts in human tissues and organs | |
|---|---|
| **Tissues** | **KANSARL** |
| adipose tissue | + |
| adrenal gland | + |
| ovary | + |
| appendix | + |
| bladder | + |
| bone marrow | - |
| cerebral cortex | + |
| colon | + |
| duodenum | + |
| endometrium | + |
| esophagus | + |
| fallopian tube | + |
| gall bladder | + |
| heart | + |
| kidney | - |
| liver | + |
| lung | + |
| lymph node | + |
| pancreas | + |
| placenta | + |
| prostate | + |
| rectum | + |
| salivary gland | + |
| skeletal muscle | + |
| skin | + |

(continued)

| Table 17 - Distribution of KARSARL fusion transcripts in human tissues and organs | |
|---|---|
| **Tissues** | **KANSARL** |
| small intestine | + |
| smooth muscle | - |
| spleen | + |
| stomach | - |
| testis | + |
| thyroid | + |
| tonsil | + |

In order to verify KANSARL fusion transcripts could be detected at such highly frequencies, we have performed RT-PCR amplification of uncharacterized samples of breast cancer cell lines and lymphomas available. Figure 7a showed that we have performed RT-PCR on 10 breast cancer cell lines and 4 of them have been found to have KANSARL isoform 2. These four KANSARL positive breast cancer cell lines are HCC-1937, T47D, MAD-436 and SUM-157, all of which have Caucasian ethnic backgrounds. Furthermore, we have performed RT-PCR amplification on 8 lymphomas cell lines. KANSARL isoform 2 has been detected in DHL-5, DHL-8, OCI-Ly10 and Val (Figure 7b) as does KANSARL isoform 1 (data not shown). Figure 7c & 7d showed that all eight lymphomas have at least one copy of KANSL1 and one copy of ARL17A gene while Figure 7e showed RT-PCR amplification of GAPHD mRNA as controls. Even though the numbers of breast cancer and lymphomas are relatively small, the percentages of KANSARL-positive cell lines are within those obtained from RNA-seq data analysis, suggesting that KANSARL fusion transcripts are highly recurrent in the cancer samples of European ancestry origin.

Figure 3 and Figure 7 show that many cancer cell lines have been shown to have dominant KANSARL isoform 2. To investigate the KANSARL isoform expression, we have performed RNA amplifications of all KANSARL isoforms on some of the KANSARL positive cell lines. Figure 8 shows that all KANSARL isoforms except for the KANSARL isoform 6 have been detected in nine KANSARL-positive cancer cell lines, including A549, Hela-3, 293T, K562, HT29, LY10, DHL-5, DHL-8 and VAL. This suggests that RT-PCR amplification can be used to detect KANSARL fusion transcripts expressed at <0.05% of the GAPHD gene expression levels.

We have demonstrated that KANSARL fusion transcripts are familial-inherited, and that KANSARL are expressed in the majorities of tissues. Supplementary Table 8 has shown that KANSARL fusion transcripts have been found in an average of 28.9% of the population of European ancestry, which ranges from 26.3% FIN to 33.7% GBR (Figure 6b). No previous evidence has suggested that KANSARL fusion transcripts are associated with cancer or are derived from cancer predisposition gene. We have provided four lines of evidence supporting that the KANSARL fusion transcripts are associated with multiple types of cancer. First, the frequency of KANSARL fusion transcripts in the CGD glioblastomas patients is significantly higher than the non-neoplastic (normal) control. Second, all KANSARL-positive prostate tumor patients also have prostate cancer biomarker TMPRSS2-ERG fusion transcripts. Third, we have shown that 4 out of 10 breast cancer cell lines and 4 out of 8 lymphoma cell lines have been detected to have KANSARL fusion transcripts. Fourth, the high frequencies of KANSARL fusion transcripts in glioblastomas, prostate, breast cancer, lung cancer and lymphomas patients from North America suggest that KANSARL fusion transcripts are associated with multiple types of cancer. Therefore, we can conclude that KANSARL fusion transcripts are derived from the cancer predisposition fusion gene.

Figure 2 has shown that six KANSARL isoforms identified encode proteins with 437, 483, 496, 505, 450 and 637 aa, majorities of which come from the KANSL1 sequences and bear similarities to some KANSL1 mutations (Koolen, Pfundt et al. 2015). KANSARL putative proteins would lack the WDR5 binding region and the Zn finger domains responsible for KAT8 activity, and PEHE domain. Loss of these domains results in KAT8 HAT inactivation to catalyze H4K16 acetylation (Huang, Wan et al. 2012), which is recently recognized as a common hallmark of human tumors (Fraga, Ballestar et al. 2005). In addition inactivation of KAT8 to catalyze p53 Lys120 acetylation inhibits the abilities of p53 to activate downstream p53 target genes, which regulate p53-mediated apoptosis and can promote cancer (Mellert, Stanek et al. 2011). Association between KANSARL and TMPRSS2-ERG fusion transcripts have been observed in prostate tumors, but not in glioblastomas or any other types of cancer analyzed so far, suggest that genomic alternations are tissue-specific and cancer-specific. Understanding these specific genetic abbreviations not only help us to develop better detection of much early stages of tumors, but also enable us to identify drug targets to block these processes. Supplementary Table 9 shows that KANSARL fusion transcripts are specifically associated with many read-through fusion transcripts, which are thought to be epigenetic. Understanding how KANSARL affect how epigcnetic alternations will result in tumor genesis. One approach is to use KANSARL-specific antibodies or siRNAs to degrade KANSARL mRNA or proteins and to check whether such degradation will restore epigenetic changes. It has of great interests to investigate whether blood transfusions from KANSARL carriers cause cancer because blood is more likely to have cancer progenitor cells and KANSARL may activate epigenetic pathways in weak patients. If cancer patients express KANSARL fusion transcripts and will reduce histone acetylation, these patients may be sensitive to histone deacetylase inhibitors (HDAC inhibitors). Therefore, typing of KANSARL fusion transcripts will improve outcomes of HDAC inhibitors.

This research has used RNA-seq datasets from diverse laboratories around the World to identify and analyze KANSARL fusion transcripts. The qualities, lengths and numbers of RNA-seq read are greatly variable from sample to sample. The main issues to analyze RNA-seq data--"Big Data" are fast and accurate. To solve both problems, we have used splicing code table and removed majorities of highly-repetitive splicing sequences from the current version of the implementation. Because our model requires that both 5' and 3' genes are present in the splicingcode table, we have greatly improved the accuracy of detecting the fusion transcripts and dramatically increased computation speeds. In addition, we have identified only fusion transcripts, whose sequences have to be identical to reference sequences. Because of these quality improvements, the maximum random error to generate a fusion transcript is $1.2 \times 10^{-24}$ and the medium error is $1 \times 10^{-59}$. Since the number of RNA-seq reads would dramatically affect detecting KANSARL fusion transcripts, especially if the samples are KANSARL negative, we have selected potential KANSARL-negative datasets with higher qualities and at least 20 million of effective RNA-seq reads. These quality controls have greatly increased data reproducibility and reduced data errors. For example, the CGD dataset has 27 glioblastoma patents, which have 39 CE samples and 36 NE samples that are effectively constituted as multiple duplication experiments. All KANSARL-positive samples have been detected in the corresponding CE and NE samples and the duplication samples and all KANSARL-negative samples are also reproducible. That is, 100% of both KANSARL-positive and KANSARL-negative samples can be reproducible. If cancer samples might contain different ethnic backgrounds, especially samples from North American may have higher possibilities of having patients from African and Asian ancestry origins, it would have some negative impacts on our data analysis. However, these minor imperfections would not affect our conclusion that KANSARL fusion transcripts are associated with cancer samples of European ancestry origin.

As shown in Figure 4, KANSARL fusion transcripts are specific to European ancestry origin and likely result from inversion of ARL17-KANSL1 genes or local duplication. The genes KANSL1, ARL17A and MAPT located in 1Mb inversion of chromosomal band 17q21.31 have been shown to have polymorphism. This inversion has resulted in the H1 and H2 haplotypes of 17q21.31, which have been shown to reach high allele frequency (26% and 19%, respectively) in West Eurasian populations, but absent in both African and Asian populations (Boettger, Handsaker et al. 2012). Analysis of genomic structures has shown that the population of European ancestry origin have short (155 kbp) and long (205 kbp) duplications corresponding to the promoter and first exon of KANSL1 associated with the H2 and H1 haplotypes, respectively (Steinberg, Antonacci et al. 2012). Both duplications have resulted in novel KANSL1 transcripts. The cDNA clone BC006271 identified in ovary adenocarcinoma (Strausberg, Feingold et al. 2002) has later been detected in one lymphoblastoid cell line of H1β population of the European ancestry origin(Boettger, Handsaker et al. 2012), and has been shown to have identical fusion junction to KANSARL isoform 2.

**Isolation of total RNAs from the cell lines.** Cell growth media were removed from the petri dishes. 1 ml of Trizol reagent(Invitrogen, CA) was added directly into the cells in the culture dishes per 10 cm$^2$ of the culture dishes. The cells were lysed directly by vortex for 15 second vigorously and the mixes were incubated at room temperature for 2-3 min. The samples were centrifuged at 4000 g for 15 minutes to separate the mixtures into a lower red, phenol-chloroform phase and a colourless upper aqueous phase. The aqueous phase was transferred to a fresh tube. The organic phase is saved if isolation of DNA or protein is desired. The RNA was precipitated by mixing with 0.5 volumes of isopropyl alcohol. After incubating samples at room temperature for 10 minutes, the RNA precipitate was pelleted by centrifuging at 12,000 g for 10 minutes at room temperature. The RNA pellet was washed twice with 1 ml of 75% ethanol and was centrifuged at 7500 g for 5 min at 4°C. The RNA pellet was air-dried at room temperature for 20 min and was dissolved in 40-80μL RAase-free water.

**Isolation of genomic DNAs from cell lines** The gemomic DNAs were isolated from A549, HeLa3 and K562 by QiagenBlood & Cell Culture DNA Mini Kit as suggested by the manufactures. In brief, 5 x 10$^6$ cells were centrifuged at 1500 x g for 10 min. After the supernatants were discarded, the cell pellets were washed twice in PBS and resuspended in PBS to a final concentration of 10$^7$ cells/ml. 0.5 ml of suspension cells were added to 1 ml of ice-cold Buffer C1 and 1.5 ml of ice-cold distilled water and mixed by inversion several time. After the mixes were incubated on ice for 10 min, the lysed cells were centrifuged at 1,300×g for 15 min. After the supernatants were discarded, the pelleted nuclei were resuspended in 0.25 ml of ice-cold Buffer C1 and 0.75 ml of ice-cold distilled water and mixed by vortexing. The nuclei were centrifuged again at 4 °C for 15 min and the supernatants were discarded. The pellets were resuspended in 1 ml of Buffer G2 by vortexing for 30 sec at the maximum speed. After adding 25 ul of proteinase K, the mixes were incubated at 50 °C for 60 min. After A Qiagen Genomic-tip G20 was equilibrated with 1 ml of Buffer QBT and emptied by gravity flow, the sample were applied to the equilibrated Genomic-tip G20 and allowed to enter resin by gravity flow. After the Genomic-tip G20 was wash by 1 ml of Buffer QC three times, the genomic DNA was eluted by 1 ml of Buffer QF twice. The eluted DNA was precipitated by adding 1.4 ml of isopropanol by mixing several times and immediately centrifuged at 5,000×g for 15 min at 4 °C. After removing the supernatants, the DNA pellet was washed by 70% of ethanol three times. After air drying for 10 min, the DNA pellet was resuspended in 0.2 ml of TE buffer to the final concentration of 0.5 ug/ul.

**cDNA Synthesis** The first-strand cDNA synthesis is carried out using oligo(T)15 and/or random hexamers by TaqMan Reverse Transcription Reagents (Applied Biosystems Inc., Foster City, CA, USA) as suggested by the manufacturer. In brief, to prepare the 2X RT master mix, we pool 10 µl of reaction mixes containing final concentrations of IX RT Buffer, 1.75 mM $MgCl_2$, 2mM dNTP mix (0.5 mM each), 5 mM DTT, 1X random primers, 1.0 U /µl RNase inhibitor and 5.0 U/µl MultiScribe RT. The master mixes are prepared, spanned down and placed on ice. 10 µl of 2XRNA mixes containing 2ug of total RNA are added into 10 µl 2X master mixes and mixed well. The reaction mixes are then placed in a thermal cycler of 25 °C, 10 min, 37 °C 120 min, 95 °C, 5 min and 4 °C, ∞. The resulted cDNAs are diluted by 80 µl of $H_2O$.

**RT-PCR amplification** To identify novel human fusion transcripts, fusion transcript specific primers have been designed to cover the 5' and 3' fusion transcripts. The primers are designed using the primer-designing software (SDG 2015). 5 µl of the cDNAs generated above are used to amplify fusion transcripts by PCR. PCR reactions have been carried out by HiFi Taq polymerase (Invitrogen, Carlsbad, CA, USA) using cycles of 94°C, 15", 60-68°C, 15" and 68°C, 2-5 min. The PCR products are separated on 2% agarose gels. The expected products are excised from gels and cloned Fusion transcripts are then verified by blast and manual inspection.

**Quantitative real-time PCR.** To quantify expression levels of different KANSARL isoforoms, The primers are designed using the primer-designing software (SDG 2015). 5 µl of the cDNAs generated above are used to amplify fusion transcripts by PCR. PCR reactions have been carried out using SYBR Green PCR Master Mix (Roche) on a LightCycler 480II system(Roche) as manufacturer suggested. For each reaction, 5ul of 480 SYBR Green I Master Mix (2X), 2 ul of primers (10X) and 3 ul of $H_2O$ were pooled into a tube and mixed carefully by pipetting up and down. 15 ul of PCR mix were pepetted into each well of the LightCycler® 480 Multiwell Plate, 5 ul of cDNA were added into the wells. The Multiwell Plate was sealed with LightCycler® 480 Multiwell sealing foil. The Plate was centrifuged at 1500 × g for 2 min and transferred into the plate holder of the LightCycler 480 Instrument. The PCR was performed for 45 amplification cycles.

**PCR amplification of genomic DNAs** 0.25 ug of human A549, HeLa3 and K562 genomic DNAs were used for PCR amplification. Genomic KANSARL fusion gene was amplified by primers KANSARLgFI (Seq ID NO.: 886,574) and KANSARLgR1 (Seq ID NO.: 886,575). PCR reactions have been carried out by HiFi Taq polymerase (Invitrogen, Carlsbad, CA, USA) using cycles of 94°C, 15", 60 °C, 15" and 68°C, 2-5 min. The PCR products are separated on 1.5% agarose gels and generate a 360 bp PCR fragments.

**Statistical analysis.** To compare two different populations, we have used the two-tailed Z score analyses to whether two populations differ significantly on the genetic characteristics. We set the null hypothesis to be that there is no difference between the two population proportions. Z scores are calculated based on the following the formula:

$$Z = \frac{(\bar{p}_1 - \bar{p}_2) - 0}{\sqrt{\bar{p}(1 - \bar{p})\left(\frac{1}{n_1} + \frac{1}{n_2}\right)}}$$

REFERENCES:

Abate, F. et al., (2015) "Distinct Viral and Mutational Spectrum of Endemic Burkitt Lymphoma" PLoS Pathog 11(10): e1005158.

Balbin, O. A. et al. (2015) "The landscape of antisense gene expression in human cancers" Genome Res 25(7): 1068-1079.

Bao, Z. S. et al. (2014), "RNA-seq of 272 gliomas revealed a novel, recurrent PTPRZ1-MET fusion transcript in secondary glioblastomas" Genome Res 24(11): 1765-1773.

Boettger, L. M. et al. (2012) "Structural haplotypes and recent evolution of the human 17q21.31 region," Nat Genet 44(8): 881-885.

Fraga, M. F. et al. (2005) "Loss of acetylation at Lys16 and trimethylation at Lys20 of histone H4 is a common hallmark of human cancer," Nat Genet 37(4): 391-400.

Genomes Project, C., A. Auton, L. D. Brooks, R. M. Durbin, E. P. Garrison, H. M. Kang, J. O. Korbel, J. L. Marchini, S. McCarthy, G. A. McVean and G. R. Abecasis (2015). "A global reference for human genetic variation," Nature 526(7571): 68-74.

Gill, B. J. et al (2014), "MRI-localized biopsies reveal subtype-specific differences in molecular and cellular composition at the margins of glioblastoma" Proc Natl Acad Sci USA 111(34): 12550-12555.

Huang, J. et al. (2012) "Structural insight into the regulation of MOF in the male-specific lethal complex and the non-specific lethal complex," Cell Res 22(6): 1078-1081.

Ju, Y. S. et al. (2012) "A transforming KIF5B and RET gene fusion in lung adenocarcinoma revealed from whole-genome and transcriptome sequencing," Genome Res 22(3): 436-445.

Kinsella, M. et al. (2011) "Sensitive gene fusion detection using ambiguously mapping RNA-Seq read pairs," Bioinformatics 27(8): 1068-1075.

Koolen, D. A. et al. (2015) "The Koolen-de Vries syndrome: a phenotypic comparison of patients with a 17q21.31 microdeletion versus a KANSL1 sequence variant," Eur J Hum Genet.

Li, X. et al. (2014). "Transcriptome sequencing of a large human family identifies the impact of rare noncoding variants." Am J Hum Genet 95(3): 245-256.

Li, X. et al. (2009). "Two mammalian MOF complexes regulate transcription activation by distinct mechanisms." Mol Cell 36(2): 290-301.

Liu, S. et al. (2015). "Comprehensive evaluation of fusion transcript detection algorithms and a meta-caller to combine top performing methods in paired-end RNA-seq data." Nucleic Acids Res.

Mellert, H. S. (2011). "Deacetylation of the DNA-binding domain regulates p53-mediated apoptosis." J Bioi Chem 286(6): 4264-4270.

Mertens, F. et al. (2015). "The emerging complexity of gene fusions in cancer." Nat Rev Cancer 15(6): 371-381.

Meunier, S. et al. (2015). "An epigenetic regulator emerges as microtubule minus-end binding and stabilizing factor in mitosis." Nat Commun 6: 7889.

Morin, R. D. et al. (2013). "Mutational and structural analysis of diffuse large B-cell lymphoma using whole-genome sequencing." Blood 122(7): 1256-1265.

Rahman, N. (2014). "Realizing the promise of cancer predisposition genes." Nature 505(7483): 302-308.

Ren, S. et al. (2012). "RNA-seq analysis of prostate cancer in the Chinese population identifies recurrent gene fusions, cancer-associated long noncoding RNAs and aberrant alternative splicings." Cell Res 22(5): 806-821.

Schmitz, R. et al. (2012). "Burkitt lymphoma pathogenesis and therapeutic targets from structural and functional genomics." Nature 490(7418): 116-120.

SDG (2015). "http://www.yeastgenome.org ".

Stadler, Z. K. et al. (2014). "Cancer genomics and inherited risk." J Clin Oncol 32(7): 687-698.

Steinberg, K. M. et al. (2012). "Structural diversity and African origin of the 17q21.31 inversion polymorphism." Nat Genet 44(8): 872-880.

Strausberg, R. L. et al. (2002). "Generation and initial analysis of more than 15,000 full-length human and mouse cDNA sequences." Proc Natl Acad Sci USA 99(26): 16899-16903.

Uhlen, M. et al. (2015). "Proteomics. Tissue-based map of the human proteome." Science 347(6220): 1260419.

Varley, K. E. et al. (2014). "Recurrent read-through fusion transcripts in breast cancer." Breast Cancer Res Treat 146(2): 287-297.

Wyatt, A. W. et al. (2014). "Heterogeneity in the inter-tumor transcriptome of high risk prostate cancer." Genome Biol 15(8): 426.

Yendamuri, S., F. Trapasso and G. A. Calin (2008). "ARLTS1 - a novel tumor suppressor gene." Cancer Lett 264(1): 11-20.

Yoshihara, K. et al. (2014). "The landscape and therapeutic relevance of cancer-associated transcript fusions." Oncogene.

Zhuo, D. et al. (2012). Decipering splicing codes of spliceosomal introns BIOCOMP 2012, Las Vagas, Nevada, USA, CSREA Press.

Zhuo, D. et. al. (2007). "Modern origin of numerous alternatively spliced human introns from tandem arrays." Proc Natl Acad Sci USA 104(3): 882-886.

## Claims

1. A kit for detecting at least one KANSARL fusion transcript from a biological sample from a subject, comprising at least one of the following components:

   (a) at least one probe, wherein each of the at least one probe comprises a sequence that hybridizes specifically to a junction of the at least one KANSARL fusion transcript, said junction comprising a nucleotide sequence as set forth in SEQ ID NO: 886,551;
   (b) at least one pair of probes, wherein each of the at least one pair of probes comprises: a firstprobe comprising a sequence that hybridizes specifically to KANSL1; and a second probe comprising a sequence that hybridizes specifically to ARL17A, each of the at least one pair of probes comprises a pair of nucleotide sequences selected from one of SEQ ID NO: 886566 and SEQ ID NO: 886567; SEQ ID NO: 886568 and SEQ ID NO:886569; or (c) at least one pair of amplification primers, wherein each of the at least one pair of amplification primers are configured to specifically amplify the at least one KANSARL fusion transcript, each of the at least one pair of amplification primers comprises a pair of nucleotide sequences selected from one of SEQ ID NO: 886566 and SEQ ID NO: 886567; SEQ ID NO: 886568 and SEQ ID NO: 886569.

2. The kit according to claim 1, further comprising compositions configured to extract a RNA sample from the biological sample, and to generate cDNA molecules from the RNA sample.

3. The kit according to claim 1, wherein the biological sample is selected from a group consisting of a cell line, buccal cells, adipose tissue, adrenal gland, ovary, appendix, bladder, bone marrow, cerebral cortex, colon, duodenum, endometrium, esophagus, fallopian tube, gall bladder, heart, kidney, liver, lung, lymph node, pancreas, placenta, prostate, rectum, salivary gland, skeletal muscle, skin, blood, small intestine, smooth muscle, spleen, stomach, testis, thyroid, and tonsil.

4. The kit according to claim 1, wherein the components as set forth in (a) comprise a plurality of probes and a substrate, wherein the plurality of probes are immobilized on the substrate.

5. The kit according to claim 1, wherein the first probe and the second probe respectively comprises a first moiety and a second moiety, configured to indicateco-hybridization of the first probe and the second probe in a hybridization reaction to thereby detect a presence of the at least one KANSARL fusion transcript.

6. A method for detecting presence or absence of at least one KANSARL fusion transcript in a biological sample from a subject utilizing the kit according to claim 1, comprising the steps of: (i) treating the biological sample to obtain a treated sample; (ii) contacting the treated sample with at least one components as set forth in (a), (b), or (c) of the kit for a reaction; and (iii) determining that the at least one KANSARL fusion transcript is present in the biological sample if the reaction generates a positive result, or that the at least one KANSARL fusion transcript is absent in the biological sample if otherwise.

7. The method according to claim 6, wherein the reaction in step (ii) is hybridization reaction.

8. The method according to claim 7, wherein the components as set forth in (b) are utilized, and the positive result in step (iii) is co-localization of the first probe and the second probe in the hybridization reaction, wherein, preferably, the hybridization reaction in step (ii) is in situ hybridization (ISH) or Northern blot.

9. The method according to claim 7, wherein the components as set forth in (a) are utilized, and the positive result in step (iii) is hybridization of the at least one probe with at least one polynucleotide in the treated sample, wherein, preferably, the treated sample in step (i) is a cDNA sample, and step (i) comprises the sub-steps of: isolating a RNA sample from the biological sample; and obtaining the cDNA sample from the RNA sample, wherein, more preferably, the hybridization reaction in step (ii) is Southern blot, dot blot, or microarray.

10. The method according to claim 6, wherein the reaction in step (ii) is amplification reaction, the components as set forth in (c) are utilized, and the positive result in step (iii) is obtaining of at least one amplified polynucleotide of expected size.

11. The method according to claim 10, wherein: each of the at least one pair of amplification primers in the components as set forth in (c) comprises a pair of nucleotide sequences selected from one of SEQ ID NO: 886566 and SEQ ID NO: 886567; SEQ ID NO: 886568 and SEQ ID NO: 886569; and the expected size of the at least one amplified polynucleotide is 431 bp or 236 bp, wherein, preferably, the first amplification primer and the second amplification primer respectively comprises a nucleotide sequence as set forth in SEQ ID NO: 886566 and SEQ ID NO: 886567 and the expected size of the amplified polynucleotide is 431 bp.

12. The method according to claim 10, wherein step (iii) further comprises verification of the at least one amplified polynucleotide by sequencing.

**Patentansprüche**

1. Kit zum Detektieren von mindestens einem KANSARL-Fusionstranskript von einer biologischen Probe von einem Individuum, umfassend mindestens einen der folgenden Bestandteile:

   (a) mindestens eine Sonde, wobei jede der mindestens einen Sonde eine Sequenz umfasst, die spezifisch an eine Verbindungsstelle des mindestens einen KANSARL-Fusionstranskripts hybridisiert, wobei die Verbindungsstelle eine wie in SEQ ID NO: 886,551 dargelegte Nukleotidsequenz umfasst;
   (b) mindestens ein Sonden-Paar, wobei jedes des mindestens einen Sonden-Paars umfasst: eine erste Sonde, die eine Sequenz umfasst, die spezifisch an KANSL1 hybridisiert; und eine zweite Sonde, die eine Sequenz umfasst, die spezifisch an ARL17A hybridisiert, wobei jedes des mindestens einen Sonden-Paars ein Nukleotidsequenzen-Paar, ausgewählt aus einem von SEQ ID NO: 886566 und SEQ ID NO: 886567; SEQ ID NO: 886568 und SEQ ID NO: 886569, umfasst; oder
   (c) mindestens ein Amplifikationsprimer-Paar, wobei jedes des mindestens einen Amplifikationsprimer-Paars zum spezifischen Amplifizieren des mindestens einen KANSARL-Fusionstranskripts konfiguriert ist, wobei jedes des mindestens einen Amplifikationsprimer-Paars ein Nukleotidsequenzen-Paar, ausgewählt aus einem von SEQ ID NO: 886566 und SEQ ID NO: 886567; SEQ ID NO: 886568 und SEQ ID NO: 886569, umfasst.

2. Kit nach Anspruch 1, weiter umfassend Zusammensetzungen, die zum Extrahieren einer RNA-Probe aus der biologischen Probe und zum Erzeugen von cDNA-Molekülen aus der RNA-Probe konfiguriert sind.

3. Kit nach Anspruch 1, wobei die biologische Probe ausgewählt ist aus einer Gruppe, bestehend aus einer Zelllinie, bukkalen Zellen, Fettgewebe, Nebenniere, Eierstock, Blinddarm, Blase, Knochenmark, Gehirnrinde, Dickdarm, Zwölffingerdarm, Endometrium, Speiseröhre, Eileiter, Gallenblase, Herz, Niere, Leber, Lunge, Lymphknoten, Bauchspeicheldrüse, Plazenta, Prostata, Rektum, Speicheldrüse, Skelettmuskel, Haut, Blut, Dünndarm, glattem Muskel, Milz, Magen, Hoden, Schilddrüse und Mandeln.

4. Kit nach Anspruch 1, wobei die in (a) dargelegten Bestandteile eine Vielzahl von Sonden und ein Substrat umfassen, wobei die Vielzahl von Sonden an dem Substrat immobilisiert sind.

5. Kit nach Anspruch 1, wobei die erste Sonde und die zweite Sonde einen ersten Teil beziehungsweise einen zweiten Teil umfassen, die zum Anzeigen einer Co-Hybridisierung der ersten Sonde und der zweiten Sonde in einer Hybridisierungsreaktion konfiguriert sind, um dadurch ein Vorhandensein des mindestens einen KANSARL-Fusionstranskripts zu detektieren.

6. Verfahren zum Detektieren eines Vorhandenseins oder einer Abwesenheit von mindestens einem KANSARL-Fusionstranskript in einer biologischen Probe von einem Individuum unter Verwendung des Kits nach Anspruch 1, umfassend die Schritte: (i) Behandeln der biologischen Probe, um eine behandelte Probe zu erhalten; (ii) In-Kontakt-Bringen der behandelten Probe mit mindestens einem der in (a), (b) oder (c) dargelegten Bestandteile des Kits für eine Reaktion; und (iii) Bestimmen, dass das mindestens eine KANSARL-Fusionstranskript in der biologischen Probe vorhanden ist, falls die Reaktion ein positives Ergebnis erzeugt, oder anderenfalls, dass das mindestens eine KANSARL-Fusionstranskript in der biologischen Probe abwesend ist.

7. Verfahren nach Anspruch 6, wobei die Reaktion in Schritt (ii) eine Hybridisierungsreaktion ist.

8. Verfahren nach Anspruch 7, wobei die in (b) dargelegten Bestandteile verwendet werden und das positive Ergebnis in Schritt (iii) eine Co-Lokalisierung der ersten Sonde und der zweiten Sonde bei der Hybridisierungsreaktion ist, wobei, bevorzugt, die Hybridisierungsreaktion in Schritt (ii) eine in situ Hybridisierung (ISH) oder ein Northern Blot ist.

9. Verfahren nach Anspruch 7, wobei die in (a) dargelegten Bestandteile verwendet werden und das positive Ergebnis in Schritt (iii) eine Hybridisierung der mindestens einen Sonde mit mindestens einem Polynukleotid in der behandelten Probe ist, wobei, bevorzugt, die behandelte Probe in Schritt (i) eine cDNA-Probe ist und Schritt (i) die Unterschritte umfasst: Isolieren einer RNA-Probe aus der biologischen Probe; und Erhalten der cDNA-Probe aus der RNA-Probe, wobei, bevorzugter, die Hybridisierungsreaktion in Schritt (ii) ein Southern Blot, Dot Blot oder Microarray ist.

10. Verfahren nach Anspruch 6, wobei die Reaktion in Schritt (ii) eine Amplifizierungsreaktion ist, die in (c) dargelegten Bestandteile verwendet werden und das positive Ergebnis in Schritt (iii) ein Erhalten von mindestens einem amplifizierten Polynukleotid einer erwarteten Größe ist.

**11.** Verfahren nach Anspruch 10, wobei: jedes des mindestens einen Amplifikationsprimer-Paars bei den in (c) dargelegten Bestandteilen ein Nukleotidsequenzen-Paar umfasst, ausgewählt aus einem von SEQ ID NO: 886566 und SEQ ID NO: 886567; SEQ ID NO: 886568 und SEQ ID NO: 886569; und die erwartete Größe des mindestens einen amplifizierten Polynukleotids 431 bp oder 236 bp ist, wobei, bevorzugt, der erste Amplifikationsprimer und der zweite Amplifikationsprimer eine wie in SEQ ID NO: 886566 beziehungsweise SEQ ID NO: 886567 dargelegte Nukleotidsequenz umfassen und die erwartete Größe des amplifizierten Polynukleotids 431 bp ist.

**12.** Verfahren nach Anspruch 10, wobei Schritt (iii) weiter ein Verifizieren des mindestens einen amplifizierten Polynukleotids durch Sequenzieren umfasst.

**Revendications**

**1.** Kit de détection d'au moins un produit de transcription de fusion de KANSARL à partir d'un échantillon biologique d'un sujet, comprenant au moins un des composants suivants :

(a) au moins une sonde, où chacune de la au moins une sonde comprend une séquence qui s'hybride spécifiquement à une jonction du au moins un produit de transcription de fusion de KANSARL, ladite jonction comprenant une séquence de nucléotides telle que décrite dans SEQ ID NO: 886 551 ;
(b) au moins une paire de sondes, où chacune de la au moins une paire de sondes comprend : une première sonde comprenant une séquence qui s'hybride spécifiquement à KANSL1 ; et une seconde sonde comprenant une séquence qui s'hybride spécifiquement à ARL17A, chacune de la au moins une paire de sondes comprend une paire de séquences de nucléotides sélectionnée parmi l'une de SEQ ID NO: 886 566 et SEQ ID NO: 886 567 ; SEQ ID NO: 886 568 et SEQ ID NO: 886 569 ; ou
(c) au moins une paire d'amorces d'amplification, où chacune de la au moins une paire d'amorces d'amplification sont configurées pour spécifiquement amplifier le au moins un produit de transcription de fusion de KANSARL, chacune de la au moins une paire d'amorces d'amplification comprend une paire de séquences de nucléotides sélectionnée parmi l'une de SEQ ID NO: 886 566 et SEQ ID NO: 886 567 ; SEQ ID NO: 886 568 et SEQ ID NO: 886 569.

**2.** Kit selon la revendication 1, comprenant en outre des compositions configurées pour extraire un échantillon d'ARN à partir de l'échantillon biologique, et pour générer des molécules d'ADNc à partir de l'échantillon d'ARN.

**3.** Kit selon la revendication 1, dans lequel l'échantillon biologique est sélectionné dans un groupe consistant en une lignée cellulaire, des cellules buccales, du tissu adipeux, une glande surrénale, un ovaire, un appendice, la vessie, la moelle osseuse, le cortex cérébral, le côlon, le duodénum, l'endomètre, l'œsophage, une trompe de Fallope, la vésicule biliaire, le cœur, le rein, le foie, le poumon, un ganglion lymphatique, le pancréas, le placenta, la prostate, le rectum, une glande salivaire, un muscle squelettique, la peau, du sang, l'intestin grêle, un muscle lisse, la rate, l'estomac, un testicule, la thyroïde, et une amygdale.

**4.** Kit selon la revendication 1, dans lequel les composants tels que décrits en (a) comprennent une pluralité de sondes et un substrat, dans lequel la pluralité de sondes sont immobilisées sur le substrat.

**5.** Kit selon la revendication 1, dans lequel la première sonde et la seconde sonde comprennent respectivement un premier fragment et un second fragment, configurés pour indiquer une co-hybridation de la première sonde et de la seconde sonde dans une réaction d'hybridation afin d'ainsi détecter une présence du au moins un produit de transcription de fusion de KANSARL.

**6.** Procédé pour détecter la présence ou l'absence d'au moins un produit de transcription de fusion de KANSARL dans un échantillon biologique d'un sujet en utilisant le kit selon la revendication 1, comprenant les étapes consistant à : (i) traiter l'échantillon biologique afin d'obtenir un échantillon traité ; (ii) mettre en contact l'échantillon traité avec au moins un des composants tels que décrits en (a), (b), ou (c) du kit pour une réaction ; et (iii) déterminer que le au moins un produit de transcription de fusion de KANSARL est présent dans l'échantillon biologique si la réaction génère un résultat positif, ou que le au moins un produit de transcription de fusion de KANSARL est absent dans l'échantillon biologique dans le cas contraire.

**7.** Procédé selon la revendication 6, dans lequel la réaction à l'étape (ii) est une réaction d'hybridation.

**8.** Procédé selon la revendication 7, dans lequel les composants tels que décrits en (b) sont utilisés, et le résultat positif à l'étape (iii) est la co-localisation de la première sonde et de la seconde sonde dans la réaction d'hybridation, dans lequel, de préférence, la réaction d'hybridation à l'étape (ii) est une hybridation *in situ* (ISH) ou un transfert de Northern.

**9.** Procédé selon la revendication 7, dans lequel les composants tels que décrits en (a) sont utilisés, et le résultat positif à l'étape (iii) est l'hybridation de la au moins une sonde avec au moins un polynucléotide dans l'échantillon traité, dans lequel, de préférence, l'échantillon traité à l'étape (i) est un échantillon d'ADNc, et l'étape (i) comprend les sous-étapes consistant à : isoler un échantillon d'ARN à partir de l'échantillon biologique ; et obtenir l'échantillon d'ADNc à partir de l'échantillon d'ARN, dans lequel, de manière davantage préférée, la réaction d'hybridation à l'étape (ii) est un transfert de Southern, une hybridation sur tache, ou un microréseau.

**10.** Procédé selon la revendication 6, dans lequel la réaction à l'étape (ii) est une réaction d'amplification, les composants tels que décrits en (c) sont utilisés, et le résultat positif à l'étape (iii) est l'obtention d'au moins un polynucléotide amplifié ayant la taille attendue.

**11.** Procédé selon la revendication 10, dans lequel : chacune de la au moins une paire d'amorces d'amplification dans les composants tels que décrits en (c) comprend une paire de séquences de nucléotides sélectionnée parmi l'une de SEQ ID NO: 886 566 et SEQ ID NO: 886 567 ; SEQ ID NO: 886 568 et SEQ ID NO: 886 569 ; et la taille attendue du au moins un polynucléotide amplifié est de 431 pb ou 236 pb, dans lequel, de préférence, la première amorce d'amplification et la seconde amorce d'amplification comprennent respectivement une séquence de nucléotides telle que décrite dans SEQ ID NO: 886 566 et SEQ ID NO: 886 567 et la taille attendue du polynucléotide amplifié est de 431 pb.

**12.** Procédé selon la revendication 10, dans lequel l'étape (iii) comprend en outre la vérification du au moins un polynucléotide amplifié par un séquençage.

ARL17A  KANSL1          KANSL1        ARL17A

LRRC37C   MAPT                              LRRC37C

## FIG. 1A

1
```
          1  2  3  4      10 11
GAG••52••TCATGTACCCCT GTTTCTGTGTGG••62••GAC
```

2
```
          1  2  3  3  4      10 11
TTC••62••CGTGCTAATAAG GTTTCTGTGTGG••62••GAC
```

3
```
          1  2  3  4  5      10 11
CAG••17••CGTGCTAATAAG GTGCCAGAAGCC••28••AGC
```

4
```
          1  2  8  9  10 11
TCT••44••TCATGTACCCCT AGCTATCAGCTTC••28••TGC
```

5
```
          1  2  3  8  9  10 11
TAC••10••CGTGCTAATAAG AGCTATCAGCTTC••17••GGG
```

6
```
          1  2    5  6  7  8  9  10 11
TGT••40••CTTTCCAAGAAG GTTCGTGAGCCA••31••GCT
```

## FIG. 1B

FIG. 1C

FIG. 1D

FIG. 1E

FIG. 2A

```
gctgatcccgagcagcgtcatgtaccccctgtttctgtgtggagacagtagaatataaaaa
 A  D  P  E  Q  R  H  V  P  L  F  L  C  G  D  S  E  I
taacaccttcgctgtctgggatgttggcagccacttcaaaatcagacctctgtggcagca
```

FIG. 2B

```
tatcgaattcgtcagcaaacagacatttacaaacagatacgtgctaataaggtttctgtg
 Y  R  I  R  Q  Q  T  D  I  Y  K  Q  I  R  A  N  K  V  S  V
tggagacagtagaatataaaaataacaccttcgctgtctgggatgttggcagccacttca
 W  R  Q  ~
```

FIG. 2C

```
tatcgaattcgtcagcaaacagacatttacaaacagatacgtgctaataaggtgccagaa
 Y  R  I  R  Q  Q  T  D  I  Y  K  Q  I  R  A  N  K  V  P  E
gcccaggaagcacacatcaaggctcacttgccagcgggatgctgccaataaaatgtagtc
 A  Q  E  A  H  I  K  A  H  L  P  A  G  C  C  Q  ~
```

FIG. 2D

```
tatcgaattcgtcagcaaacagacatttacaaacagatacgtgctaataagagctatcag
 Y  R  I  R  Q  Q  T  D  I  Y  K  Q  I  R  A  N  K  S  Y  Q
cttcccagtttgcacaattcatcaagaaattatgcgggtcaccggcacaaatgatgagg
 L  P  S  L  H  S  S  R  K  Y  A  G  S  P  A  Q  M  M
catctcctggaagcttaacttcttatccatcccatctcttggacagatgatgccagttaa
 H  L  L  E  A  ~
```

FIG. 2E

```
gctgatcccgagcagcgtcatgtaccccctagctatcagcttcccagtttgcacaattcat
 A  D  P  E  Q  R  H  V  P  L  A  I  S  F  P  V  C  T  I  H
caagaaattatgcgggtcaccggcacaaatgatgaggcatctcctggaagcttaacttc
 Q  E  I  M  R  G  H  R  H  K  ~
```

FIG. 2F

```
aagcggaggcttgttcgacccaacagcatcgttcctctttccaagaaggttcgtgagcca
 K  R  R  L  V  R  P  N  S  I  V  P  L  S  K  K  V  R  E  P
cacatctccccacaccaagctcctccatacaagacctcggactgcatcacgtaaatgctt
 H  I  S  P  H  Q  A  P  P  Y  K  T  S  D  C  I  T  ~
tttcagggggcaaaatctagagaatctgaaatggtgagcctttttcctt
```

FIG. 3A

FIG. 3B

FIG. 3C

FIG. 3D

FIG. 3E

FIG. 3F

## FIG. 3G

## FIG. 3H

## FIG. 3I

FIG. 4A

FIG. 4B

FIG. 4C

FIG. 4D

FIG. 4E

FIG. 4F

FIG. 4G

FIG. 5A

FIG. 5B

FIG. 5C

FIG. 6A

FIG. 6B

FIG. 7A

FIG. 7B

FIG. 7C

FIG. 7D

FIG. 7E

FIG. 8A    KANSARL Iso1

FIG. 8B    KANSARL Iso2

FIG. 8C    KANSARL Iso3

FIG. 8D    KANSARL Iso4

FIG. 8E    KANSARL Iso5

FIG. 8F    KANSARL Iso6

FIG. 8G    GAPDH

EP 3 475 448 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016078168 A
- US 20160078168 A1

- US 79261315

**Non-patent literature cited in the description**

- **ABATE, F. et al.** Distinct Viral and Mutational Spectrum of Endemic Burkitt Lymphoma. *PLoS Pathog,* 2015, vol. 11 (10), e1005158
- **BALBIN, O. A. et al.** The landscape of antisense gene expression in human cancers. *Genome Res,* 2015, vol. 25 (7), 1068-1079
- **BAO, Z. S. et al.** RNA-seq of 272 gliomas revealed a novel, recurrent PTPRZ1-MET fusion transcript in secondary glioblastomas. *Genome Res,* 2014, vol. 24 (11), 1765-1773
- **BOETTGER, L. M. et al.** Structural haplotypes and recent evolution of the human 17q21.31 region. *Nat Genet,* 2012, vol. 44 (8), 881-885
- **FRAGA, M. F. et al.** Loss of acetylation at Lys16 and trimethylation at Lys20 of histone H4 is a common hallmark of human cancer. *Nat Genet,* 2005, vol. 37 (4), 391-400
- **C., A. AUTON ; L. D. BROOKS ; R. M. DURBIN ; E. P. GARRISON ; H. M. KANG ; J. O. KORBEL ; J. L. MARCHINI ; S. MCCARTHY ; G. A. MCVEAN ; G. R. ABECASIS.** A global reference for human genetic variation. *Nature,* 2015, vol. 526 (7571), 68-74
- **GILL, B. J. et al.** MRI-localized biopsies reveal subtype-specific differences in molecular and cellular composition at the margins of glioblastoma. *Proc Natl Acad Sci USA,* 2014, vol. 111 (34), 12550-12555
- **HUANG, J. et al.** Structural insight into the regulation of MOF in the male-specific lethal complex and the non-specific lethal complex. *Cell Res,* 2012, vol. 22 (6), 1078-1081
- **JU, Y. S. et al.** A transforming KIF5B and RET gene fusion in lung adenocarcinoma revealed from whole-genome and transcriptome sequencing. *Genome Res,* 2012, vol. 22 (3), 436-445
- **KINSELLA, M. et al.** Sensitive gene fusion detection using ambiguously mapping RNA-Seq read pairs. *Bioinformatics,* 2011, vol. 27 (8), 1068-1075
- **KOOLEN, D. A. et al.** The Koolen-de Vries syndrome: a phenotypic comparison of patients with a 17q21.31 microdeletion versus a KANSL1 sequence variant. *Eur J Hum Genet.,* 2015
- **LI, X. et al.** Transcriptome sequencing of a large human family identifies the impact of rare noncoding variants. *Am J Hum Genet,* 2014, vol. 95 (3), 245-256
- **LI, X. et al.** Two mammalian MOF complexes regulate transcription activation by distinct mechanisms. *Mol Cell,* 2009, vol. 36 (2), 290-301
- **LIU, S. et al.** Comprehensive evaluation of fusion transcript detection algorithms and a meta-caller to combine top performing methods in paired-end RNA-seq data. *Nucleic Acids Res.,* 2015
- **MELLERT, H. S.** Deacetylation of the DNA-binding domain regulates p53-mediated apoptosis. *J Bioi Chem,* 2011, vol. 286 (6), 4264-4270
- **MERTENS, F. et al.** The emerging complexity of gene fusions in cancer. *Nat Rev Cancer,* 2015, vol. 15 (6), 371-381
- **MEUNIER, S. et al.** An epigenetic regulator emerges as microtubule minus-end binding and stabilizing factor in mitosis. *Nat Commun,* 2015, vol. 6, 7889
- **MORIN, R. D. et al.** Mutational and structural analysis of diffuse large B-cell lymphoma using whole-genome sequencing. *Blood,* 2013, vol. 122 (7), 1256-1265
- **RAHMAN, N.** Realizing the promise of cancer predisposition genes. *Nature,* 2014, vol. 505 (7483), 302-308
- **REN, S. et al.** RNA-seq analysis of prostate cancer in the Chinese population identifies recurrent gene fusions, cancer-associated long noncoding RNAs and aberrant alternative splicings. *Cell Res,* 2012, vol. 22 (5), 806-821
- **SCHMITZ, R. et al.** Burkitt lymphoma pathogenesis and therapeutic targets from structural and functional genomics. *Nature,* 2012, vol. 490 (7418), 116-120
- **STADLER, Z. K. et al.** Cancer genomics and inherited risk. *J Clin Oncol,* 2014, vol. 32 (7), 687-698
- **STEINBERG, K. M. et al.** Structural diversity and African origin of the 17q21.31 inversion polymorphism. *Nat Genet,* 2012, vol. 44 (8), 872-880

- **STRAUSBERG, R. L. et al.** Generation and initial analysis of more than 15,000 full-length human and mouse cDNA sequences. *Proc Natl Acad Sci USA,* 2002, vol. 99 (26), 16899-16903
- **UHLEN, M. et al.** Proteomics. Tissue-based map of the human proteome. *Science,* 2015, vol. 347 (6220), 1260419
- **VARLEY, K. E. et al.** Recurrent read-through fusion transcripts in breast cancer. *Breast Cancer Res Treat,* 2014, vol. 146 (2), 287-297
- **WYATT, A. W. et al.** Heterogeneity in the inter-tumor transcriptome of high risk prostate cancer. *Genome Biol,* 2014, vol. 15 (8), 426
- **YENDAMURI ; S., F. TRAPASSO ; G. A. CALIN.** ARLTS1 - a novel tumor suppressor gene. *Cancer Lett,* 2008, vol. 264 (1), 11-20
- **YOSHIHARA, K. et al.** The landscape and therapeutic relevance of cancer-associated transcript fusions. *Oncogene,* 2014
- Decipering splicing codes of spliceosomal introns. **ZHUO, D. et al.** BIOCOMP 2012. CSREA Press, 2012
- **ZHUO, D.** Modern origin of numerous alternatively spliced human introns from tandem arrays. *Proc Natl Acad Sci USA,* 2007, vol. 104 (3), 882-886